# EUROPEAN PATENT SPECIFICATION

(11) **EP 1 029 069 B1**
(45) Date of publication and mention of the grant of the patent: **26.07.2006**
(21) Application number: 98954098.4
(22) Date of filing: 13.11.1998
(51) Int. Cl.: C12N 15/86

(54) **ALPHAVIRUS VECTORS**
ALPHAVIRUS-VEKTOREN
VECTEURS D'ALPHAVIRUS

(30) Priority: 14.11.1997 US 65793 P
(43) Date of publication of application: 23.08.2000
(73) Proprietor: Sanofi Pasteur Limited, Toronto, Ontario M2R 3T4 (CA)
(72) Inventor: PARRINGTON, Mark, Bradford, Ontario L3Z 1Z4 (CA); KLEIN, Michel, Willowdale, Ontario M2P 1B9 (CA)
(74) Representative: Bizley, Richard Edward
(86) International application number: PCT/CA1998/001065
(87) International publication number: WO 1999/025859

(56) References cited:
- WO-A-95/27044
- WO-A-96/17072
- WO-A-96/40945
- ZHOU X. ET AL.: "Self-replicating Semliki-Forest virus RNA as recombinant vaccine" VACCINE, vol. 12, no. 16, 1994, pages 1510-1514, XP002089524 cited in the application
- LILJESTROEM P. ET AL.: "A NEW GENERATION OF ANIMAL CELL EXPRESSION VECTORS BASED ON THE SEMLIKI FOREST VIRUS REPLICON" BIO/TECHNOLOGY, vol. 9, December 1991, pages 1356-1361, XP000616021 cited in the application

### SEQUENCE LISTING

<110> Parrington, Mark
   Connaugnt Laboratories Limited et al, Mark
<120> ALPAHVIRUS VECTORS
<130> 862
<140> PCT/CA98/01065
   <141> 1998-11-13
<160> 12
<170> PatentIn ver. 2.0
<210> 1
   <211> 576
   <212> DNA
   <213> Semliki Forest virus
<220>
<400> 1
   gtgagtttgg ggacccttga ttgttctttc tttttcgcta ttgtaaaatt catgttatat 60
   ggagggggca aagttttcag ggtgttgttt agaatgggaa gatgtccctt gtatcaccat 120
   ggaccctcat gataattttg tttctttcac tttctactct gttgacaacc attgtctcct 180
   cttattttct tttcattttc tgtaactttt tcgttaaact ttagcttgca tttgtaacga 240
   atttttaaat tcacttttgt ttatttgtca gattgtaagt actttctcta atcacttttt 300
   tttcaaggca atcagggtat attatattgt acttcagcac agttttagag aacaattgtt 360
   ataattaaat gataaggtag aatatttctg catataaatt ctggctggcg tggaaatatt 420
   cttattggta gaaacaacta catcctggtc atcatcctgc ctttctcttt atggttacaa 480
   tgatatacac tgtttgagat gaggataaaa tactctgagt ccaaaccggg cccctctgct 540
   aaccatgttc atgccttctt ctttttccta caggtc                           576
<210> 2
   <211> 573
   <212> DNA
   <213> Semliki Forest virus
<220>
<400> 2
   gtgagtttgg ggacccttga ttgttctttc tttttcgcta ttgtaaaatt catgttatat 60
   ggagggggca aagttttcag ggtgttgttt agaatgggaa gatgtccctt gtatcaccat 120
   ggaccctcat gataattttg tttctttcac tttctactct gttgacaacc attgtctcct 180
   cttattttct tttcattttc tgtaactttt tcgttaaact ttagcttgca tttgtaacga 240
   atttttaaat tcacttttgt ttatttgtca gattgtaagt actttctcta atcacttttt 300
   tttcaaggca atcagggtat attatattgt acttcagcac agttttagag aacaattgtt 360
   ataattaaat gataaggtag aatatttctg catataaatt ctggctggcg tggaaatatt 420
   cttattggta gaaacaacta catcctggtc atcatcctgc ctttctcttt atggttacaa 480
   tgatatacac tgtttgagat gaggataaaa tactctgagt ccaaaccggg cccctctgct 540
   aaccatgttc atgccttctt ctttttccta cag                              573
<210> 3
   <211> 8010
   <212> DNA
   <213> Semliki Forest virus
<220>
<400> 3
   atcggcagtg cgccttccag gagaatgatg tctacgcaca aataccactg cgtatgccct 60
   atgcgcagcg cagaagaccc cgaaaggctc gatagctacg caaagaaact ggcagcggcc 120
   tccgggaagg tgctggatag agagatcgca ggaaaaatca ccgacctgca gaccgtcatg 180
   gctacgccag acgctgaatc tcctaccttt tgcctgcata cagacgtcac gtgtcgtacg 240
   gcagccgaag tggccgtata ccaggacgtg tatgctgtac atgcaccaac atcgctgtac 300
   catcaggcga tgaaaggtgt cagaacggcg tattggattg ggtttgacac caccccgttt 360
   atgtttgacg cgctagcagg cgcgtatcca acctacgcca caaactgggc cgacgagcag 420
   gtgttacagg ccaggaacat aggactgtgt gcagcatcct tgactgaggg aagactcggc 480
   aaactgtcca ttctccgcaa gaagcaattg aaaccttgcg acacagtcat gttctcggta 540
   ggatctacat tgtacactga gagcagaaag ctactgagga gctggcactt accctccgta 600
   ttccacctga aaggtaaaca atcctttacc tgtaggtgcg ataccatcgt atcatgtgaa 660
   gggtacgtag ttaagaaaat cactatgtgc cccggcctgt acggtaaaac ggtagggtac 720
   gccgtgacgt atcacgcgga gggattccta gtgtgcaaga ccacagacac tgtcaaagga 780
   gaaagagtct cattccctgt atgcacctac gtcccctcaa ccatctgtga tcaaatgact 840
   ggcatactag cgaccgacgt cacaccggag gacgcacaga agttgttagt gggattgaat 900
   cagaggatag ttgtgaacgg aagaacacag cgaaacacta acacgatgaa gaactatctg 960
   cttccgattg tggccgtcgc atttagcaag tgggcgaggg aatacaaggc agaccttgat 1020
   gatgaaaaac ctctgggtgt ccgagagagg tcacttactt gctgctgctt gtgggcattt 1080
   aaaacgagga agatgcacac catgtacaag aaaccagaca cccagacaat agtgaaggtg 1140
   ccttcagagt ttaactcgtt cgtcatcccg agcctatggt ctacaggcct cgcaatccca 1200
   gtcagatcac gcattaagat gcttttggcc aagaagacca agcgagagtt aatacctgtt 1260
   ctcgacgcgt cgtcagccag ggatgctgaa caagaggaga aggagaggtt ggaggccgag 1320
   ctgactagag aagccttacc acccctcgtc cccatcgcgc cggcggagac gggagtcgtc 1380
   gacgtcgacg ttgaagaact agagtatcac gcaggtgcag ggtcgtggaa aacacctcgc 1440
   agcgcgttga aagtcaccgc acagccgaac gacgtactac taggaaatta cgtagttctg 1500
   tccccgcaga ccgtgctcaa gagctccaag ttggcccccg tgcaccctct agcagagcag 1560
   gtgaaaataa taacacataa cgggagggcc ggcggttacc aggtcgacgg atatgacggc 1620
   agggtcctac taccatgtgg atcggccatt ccggtccctg agtttcaagc tttgagcgag 1680
   agcgccacta tggtgtacaa cgaaagggag ttcgtcaaca ggaaactata ccatattgcc 1740
   gttcacggac cgtcgctgaa caccgacgag gagaactacg agaaagtcag agctgaaaga 1800
   actgacgccg agtacgtgtt cgacgtagat aaaaaatgct gcgtcaagag agaggaagcg 1860
   tcgggtttgg tgttggtggg agagctaacc aaccccccgt tccatgaatt cgcctacgaa 1920
   gggctgaaga tcaggccgtc ggcaccatat aagactacag tagtaggagt ctttggggtt 1980
   ccgggatcag gcaagtctgc tattattaag agcctcgtga ccaaacacga tctggtcacc 2040
   agcggcaaga aggagaactg ccaggaaata gttaacgacg tgaagaagca ccgcgggaag 2100
   gggacaagta gggaaaacag tgactccatc ctgctaaacg ggtgtcgtcg tgccgtggac 2160
   atcctatatg tggacgaggc tttcgcttgc cattccggta ctctgctggc cctaattgct 2220
   cttgttaaac ctcggagcaa agtggtgtta tgcggagacc ccaagcaatg cggattcttc 2280
   aatatgatgc agcttaaggt gaacttcaac cacaacatct gcactgaagt atgtcataaa 2340
   agtatatcca gacgttgcac gcgtccagtc acggccatcg tgtctacgtt gcactacgga 2400
   ggcaagatgc gcacgaccaa cccgtgcaac aaacccataa tcatagacac cacaggacag 2460
   accaagccca agccaggaga catcgtgtta acatgcttcc gaggctgggc aaagcagctg 2520
   cagttggact accgtggaca cgaagtcatg acagcagcag catctcaggg cctcacccgc 2580
   aaaggggtat acgccgtaag gcagaaggtg aatgaaaatc ccttgtatgc ccctgcgtcg 2640
   gagcacgtga atgtactgct gacgcgcact gaggataggc tggtgtggaa aacgctggcc 2700
   ggcgatccct ggattaaggt cctatcaaac attccacagg gtaactttac ggccacattg 2760
   gaagaatggc aagaagaaca cgacaaaata atgaaggtga ttgaaggacc ggctgcgcct 2820
   gtggacgcgt tccagaacaa agcgaacgtg tgttgggcga aaagcctggt gcctgtcctg 2880
   gacactgccg gaatcagatt gacagcagag gagtggagca ccataattac agcatttaag 2940
   gaggacagag cttactctcc agtggtggcc ttgaatgaaa tttgcaccaa gtactatgga 3000
   gttgacctgg acagtggcct gttttctgcc ccgaaggtgt ccctgtatta cgagaacaac 3060
   cactgggata acagacctgg tggaaggatg tatggattca atgccgcaac agctgccagg 3120
   ctggaagcta gacatacctt cctgaagggg cagtggcata cgggcaagca ggcagttatc 3180
   gcagaaagaa aaatccaacc gctttctgtg ctggacaatg taattcctat caaccgcagg 3240
   ctgccgcacg ccctggtggc tgagtacaag acggttaaag gcagtagggt tgagtggctg 3300
   gtcaataaag taagagggta ccacgtcctg ctggtgagtg agtacaacct ggctttgcct 3360
   cgacgcaggg tcacttggtt gtcaccgctg aatgtcacag gcgccgatag gtgctacgac 3420
   ctaagtttag gactgccggc tgacgccggc aggttcgact tggtctttgt gaacattcac 3480
   acggaattca gaatccacca ctaccagcag tgtgtcgacc acgccatgaa gctgcagatg 3540
   cttgggggag atgcgctacg actgctaaaa cccggcggca tcttgatgag agcttacgga 3600
   tacgccgata aaatcagcga agccgttgtt tcctccttaa gcagaaagtt ctcgtctgca 3660
   agagtgttgc gcccggattg tgtcaccagc aatacagaag tgttcttgct gttctccaac 3720
   tttgacaacg gaaagagacc ctctacgcta caccagatga ataccaagct gagtgccgtg 3780
   tatgccggag aagccatgca cacggccggg tgtgcaccat cctacagagt taagagagca 3840
   gacatagcca cgtgcacaga agcggctgtg gttaacgcag ctaacgcccg tggaactgta 3900
   ggggatggcg tatgcagggc cgtggcgaag aaatggccgt cagcctttaa gggagcagca 3960
   acaccagtgg gcacaattaa aacagtcatg tgcggctcgt accccgtcat ccacgctgta 4020
   gcgcctaatt tctctgccac gactgaagcg gaaggggacc gcgaattggc cgctgtctac 4080
   cgggcagtgg ccgccgaagt aaacagactg tcactgagca gcgtagccat cccgctgctg 4140
   tccacaggag tgttcagcgg cggaagagat aggctgcagc aatccctcaa ccatctattc 4200
   acagcaatgg acgccacgga cgctgacgtg accatctact gcagagacaa aagttgggag 4260
   aagaaaatcc aggaagccat tgacatgagg acggctgtgg agttgctcaa tgatgacgtg 4320
   gagctgacca cagacttggt gagagtgcac ccggacagca gcctggtggg tcgtaagggc 4380
   tacagtacca ctgacgggtc gctgtactcg tactttgaag gtacgaaatt caaccaggct 4440
   gctattgata tggcagagat actgacgttg tggcccagac tgcaagaggc aaacgaacag 4500
   atatgcctat acgcgctggg cgaaacaatg gacaacatca gatccaaatg tccggtgaac 4560
   gattccgatt catcaacacc tcccaggaca gtgccctgcc tgtgccgcta cgcaatgaca 4620
   gcagaacgga tcgcccgcct taggtcacac caagttaaaa gcatggtggt ttgctcatct 4680
   tttcccctcc cgaaatacca tgtagatggg gtgcagaagg taaagtgcga gaaggttctc 4740
   ctgttcgacc cgacggtacc ttcagtggtt agtccgcgga agtatgccgc atctacgacg 4800
   gaccactcag atcggtcgtt acgagggttt gacttggact ggaccaccga ctcgtcttcc 4860
   actgccagcg ataccatgtc gctacccagt ttgcagtcgt gtgacatcga ctcgatctac 4920
   gagccaatgg ctcccatagt agtgacggct gacgtacacc ctgaacccgc aggcatcgcg 4980
   gacctggcgg cagatgtgca ccctgaaccc gcagaccatg tggacctcga gaacccgatt 5040
   cctccaccgc gcccgaagag agctgcatac cttgcctccc gcgcggcgga gcgaccggtg 5100
   ccggcgccga gaaagccgac gcctgcccca aggactgcgt ttaggaacaa gctgcctttg 5160
   acgttcggcg actttgacga gcacgaggtc gatgcgttgg cctccgggat tactttcgga 5220
   gacttcgacg acgtcctgcg actaggccgc gcgggtgcat atattttctc ctcggacact 5280
   ggcagcggac atttacaaca aaaatccgtt aggcagcaca atctccagtg cgcacaactg 5340
   gatgcggtcc aggaggagaa aatgtacccg ccaaaattgg atactgagag ggagaagctg 5400
   ttgctgctga aaatgcagat gcacccatcg gaggctaata agagtcgata ccagtctcgc 5460
   aaagtggaga acatgaaagc cacggtggtg gacaggctca catcgggggc cagattgtac 5520
   acgggagcgg acgtaggccg cataccaaca tacgcggttc ggtacccccg ccccgtgtac 5580
   tcccctaccg tgatcgaaag attctcaagc cccgatgtag caatcgcagc gtgcaacgaa 5640
   tacctatcca gaaattaccc aacagtggcg tcgtaccaga taacagatga atacgacgca 5700
   tacttggaca tggttgacgg gtcggatagt tgcttggaca gagcgacatt ctgcccggcg 5760
   aagctccggt gctacccgaa acatcatgcg taccaccagc cgactgtacg cagtgccgtc 5820
   ccgtcaccct ttcagaacac actacagaac gtgctagcgg ccgccaccaa gagaaactgc 5880
   aacgtcacgc aaatgcgaga actacccacc atggactcgg cagtgttcaa cgtggagtgc 5940
   ttcaagcgct atgcctgctc cggagaatat tgggaagaat atgctaaaca acctatccgg 6000
   ataaccactg agaacatcac tacctatgtg accaaattga aaggcccgaa agctgctgcc 6060
   ttgttcgcta agacccacaa cttggttccg ctgcaggagg ttcccatgga cagattcacg 6120
   gtcgacatga aacgagatgt caaagtcact ccagggacga aacacacaga ggaaagaccc 6180
   aaagtccagg taattcaagc agcggagcca ttggcgaccg cttacctgtg cggcatccac 6240
   agggaattag taaggagact aaatgctgtg ttacgcccta acgtgcacac attgtttgat 6300
   atgtcggccg aagactttga cgcgatcatc gcctctcact tccacccagg agacccggtt 6360
   ctagagacgg acattgcatc attcgacaaa agccaggacg actccttggc tcttacaggt 6420
   ttaatgatcc tcgaagatct aggggtggat cagtacctgc tggacttgat cgaggcagcc 6480
   tttggggaaa tatccagctg tcacctacca actggcacgc gcttcaagtt cggagctatg 6540
   atgaaatcgg gcatgtttct gactttgttt attaacactg ttttgaacat caccatagca 6600
   agcagggtac tggagcagag actcactgac tccgcctgtg cggccttcat cggcgacgac 6660
   aacatcgttc acggagtgat ctccgacaag ctgatggcgg agaggtgcgc gtcgtgggtc 6720
   aacatggagg tgaagatcat tgacgctgtc atgggcgaaa aacccccata tttttgtggg 6780
   ggattcatag tttttgacag cgtcacacag accgcctgcc gtgtttcaga cccacttaag 6840
   cgcctgttca agttgggtaa gccgctaaca gctgaagaca agcaggacga agacaggcga 6900
   cgagcactga gtgacgaggt tagcaagtgg ttccggacag gcttgggggc cgaactggag 6960
   gtggcactaa catctaggta tgaggtagag ggctgcaaaa gtatcctcat agccatggcc 7020
   accttggcga gggacattaa ggcgtttaag aaattgagag gacctgttat acacctctac 7080
   ggcggtccta gattggtgcg ttaatacaca gaattctgat tggatcatag cgcactatta 7140
   taggatccag atcccgggta attaattgaa ttacatccct acgcaaacgt tttacggccg 7200
   ccggtggcgc ccgcgcccgg cggcccgtcc ttggccgttg caggccactc cggtggctcc 7260
   cgtcgtcccc gacttccagg cccagcagat gcagcaactc atcagcgccg taaatgcgct 7320
   gacaatgaga cagaacgcaa ttgctcctgc taggcctccc aaaccaaaga agaagaagac 7380
   aaccaaacca aagccgaaaa cgcagcccaa gaagatcaac ggaaaaacgc agcagcaaaa 7440
   gaagaaagac aagcaagccg acaagaagaa gaagaaaccc ggaaaaagag aaagaatgtg 7500
   catgaagatt gaaaatgact gtatcttcgt atgcggctag ccacagtaac gtagtgtttc 7560
   cagacatgtc gggcaccgca ctatcatggg tgcagaaaat ctcgggtggt ctgggggcct 7620
   tcgcaatcgg cgctatcctg gtgctggttg tggtcacttg cattgggctc cgcagataag 7680
   ttagggtagg caatggcatt gatatagcaa gaaaattgaa aacagaaaaa gttagggtaa 7740
   gcaatggcat ataaccataa ctgtataact tgtaacaaag cgcaacaaga cctgcgcaat 7800
   tggccccgtg gttcgcctca cggaaactcg gggcaactca tattgacaca ttaattggca 7860
   ataattggaa gcttacataa gcttaattcg acgaataatt ggatttttat tttattttgc 7920
   aattggtttt taatatttcc aaaaaaaaaa aaaaaaaaaa aaaaaaaaaa aaaaaaaaaa 7980
   aaaaaaaaaa aaaaaaaaaa aaaaaaaaaa 8010
<210> 4
   <211> 11060
   <212> DNA
   <213> Semliki Forest virus
<220>
<400> 4
   gatggcggat gtgtgacata cacgacgcca aaagattttg ttccagctcc tgccacctcc 60
   gctacgcgag agattaacca cccacgatgg ccgccaaagt gcatgttgat attgaggctg 120
   acagcccatt catcaagtct ttgcagaagg catttccgtc gttcgaggtg gagtcattgc 180
   aggtcacacc aaatgaccat gcaaatgcca gagcattttc gcacctggct accaaattga 240
   tcgagcagga gactgacaaa gacacactca tcttggatat cggcagtgcg ccttccagga 300
   gaatgatgtc tacgcacaaa taccactgcg tatgccctat gcgcagcgca gaagaccccg 360
   aaaggctcga tagctacgca aagaaactgg cagcggcctc cgggaaggtg ctggatagag 420
   agatcgcagg aaaaatcacc gacctgcaga ccgtcatggc tacgccagac gctgaatctc 480
   ctaccttttg cctgcataca gacgtcacgt gtcgtacggc agccgaagtg gccgtatacc 540
   aggacgtgta tgctgtacat gcaccaacat cgctgtacca tcaggcgatg aaaggtgtca 600
   gaacggcgta ttggattggg tttgacacca ccccgtttat gtttgacgcg ctagcaggcg 660
   cgtatccaac ctacgccaca aactgggccg acgagcaggt gttacaggcc aggaacatag 720
   gactgtgtgc agcatccttg actgagggaa gactcggcaa actgtccatt ctccgcaaga 780
   agcaattgaa accttgcgac acagtcatgt tctcggtagg atctacattg tacactgaga 840
   gcagaaagct actgaggagc tggcacttac cctccgtatt ccacctgaaa ggtaaacaat 900
   cctttacctg taggtgcgat accatcgtat catgtgaagg gtacgtagtt aagaaaatca 960
   ctatgtgccc cggcctgtac ggtaaaacgg tagggtacgc cgtgacgtat cacgcggagg 1020
   gattcctagt gtgcaagacc acagacactg tcaaaggaga aagagtctca ttccctgtat 1080
   gcacctacgt cccctcaacc atctgtgatc aaatgactgg catactagcg accgacgtca 1140
   caccggagga cgcacagaag ctgttagtgg gattgaatca gaggatagtt gtgaacggaa 1200
   gaacacagcg aaacactaac acgatgaaga actatctgct tccgattgtg gccgtcgcat 1260
   ttagcaagtg ggcgagggaa tacaaggcag accttgatga tgaaaaacct ctgggtgtcc 1320
   gagagaggtc acttacttgc tgctgcttgt gggcatttaa aacgaggaag atgcacacca 1380
   tgtacaagaa accagacacc cagacaatag tgaaggtgcc ttcagagttt aactcgttcg 1440
   tcatcccgag cctatggtct acaggcctcg caatcccagt cagatcacgc attaagatgc 1500
   ttttggccaa gaagaccaag cgagagttaa tacctgttct cgacgcgtcg tcagccaggg 1560
   atgctgaaca agaggagaag gagaggttgg aggccgagct gactagagaa gccttaccac 1620
   ccctcgtccc catcgcgccg gcggagacgg gagtcgtcga cgtcgacgtt gaagaactag 1680
   agtatcacgc aggtgcaggg gtcgtggaaa cacctcgcag cgcgttgaaa gtcaccgcac 1740
   agccgaacga cgtactacta ggaaattacg tagttctgtc cccgcagacc gtgctcaaga 1800
   gctccaagtt ggcccccgtg caccctctag cagagcaggt gaaaataata acacataacg 1860
   ggagggccgg cggttaccag gtcgacggat atgacggcag ggtcctacta ccatgtggat 1920
   cggccattcc ggtccctgag tttcaagctt tgagcgagag cgccactatg gtgtacaacg 1980
   aaagggagtt cgtcaacagg aaactatacc atattgccgt tcacggaccg tcgctgaaca 2040
   ccgacgagga gaactacgag aaagtcagag ctgaaagaac tgacgccgag tacgtgttcg 2100
   acgtagataa aaaatgctgc gtcaagagag aggaagcgtc gggtttggtg ttggtgggag 2160
   agctaaccaa ccccccgttc catgaattcg cctacgaagg gctgaagatc aggccgtcgg 2220
   caccatataa gactacagta gtaggagtct ttggggttcc gggatcaggc aagtctgcta 2280
   ttattaagag cctcgtgacc aaacacgatc tggtcaccag cggcaagaag gagaactgcc 2340
   aggaaatagt taacgacgtg aagaagcacc gcgggaaggg gacaagtagg gaaaacagtg 2400
   actccatcct gctaaacggg tgtcgtcgtg ccgtggacat cctatatgtg gacgaggctt 2460
   tcgcttgcca ttccggtact ctgctggccc taattgctct tgttaaacct cggagcaaag 2520
   tggtgttatg cggagacccc aagcaatgcg gattcttcaa tatgatgcag cttaaggtga 2580
   acttcaacca caacatctgc actgaagtat gtcataaaag tatatccaga cgttgcacgc 2640
   gtccagtcac ggccatcgtg tctacgttgc actacggagg caagatgcgc acgaccaacc 2700
   cgtgcaacaa acccataatc atagacacca caggacagac caagcccaag ccaggagaca 2760
   tcgtgttaac atgcttccga ggctgggcaa agcagctgca gttggactac cgtggacacg 2820
   aagtcatgac agcagcagca tctcagggcc tcacccgcaa aggggtatac gccgtaaggc 2880
   agaaggtgaa tgaaaatccc ttgtatgccc ctgcgtcgga gcacgtgaat gtactgctga 2940
   cgcgcactga ggataggctg gtgtggaaaa cgctggccgg cgatccctgg attaaggtcc 3000
   tatcaaacat tccacagggt aactttacgg ccacattgga agaatggcaa gaagaacacg 3060
   acaaaataat gaaggtgatt gaaggaccgg ctgcgcctgt ggacgcgttc cagaacaaag 3120
   cgaacgtgtg ttgggcgaaa agcctggtgc ctgtcctgga cactgccgga atcagattga 3180
   cagcagagga gtggagcacc ataattacag catttaagga ggacagagct tactctccag 3240
   tggtggcctt gaatgaaatt tgcaccaagt actatggagt tgacctggac agtggcctgt 3300
   tttctgcccc gaaggtgtcc ctgtattacg agaacaacca ctgggataac agacctggtg 3360
   gaaggatgta tggattcaat gccgcaacag ctgccaggct ggaagctaga cataccttcc 3420
   tgaaggggca gtggcatacg ggcaagcagg cagttatcgc agaaagaaaa atccaaccgc 3480
   tttctgtgct ggacaatgta attcctatca accgcaggct gccgcacgcc ctggtggctg 3540
   agtacaagac ggttaaaggc agtagggttg agtggctggt caataaagta agagggtacc 3600
   acgtcctgct ggtgagtgag tacaacctgg ctttgcctcg acgcagggtc acttggttgt 3660
   caccgctgaa tgtcacaggc gccgataggt gctacgacct aagtttagga ctgccggctg 3720
   acgccggcag gttcgacttg gtctttgtga acattcacac ggaattcaga atccaccact 3780
   accagcagtg tgtcgaccac gccatgaagc tgcagatgct tgggggagat gcgctacgac 3840
   tgctaaaacc cggcggcatc ttgatgagag cttacggata cgccgataaa atcagcgaag 3900
   ccgttgtttc ctccttaagc agaaagttct cgtctgcaag agtgttgcgc ccggattgtg 3960
   tcaccagcaa tacagaagtg ttcttgctgt tctccaactt tgacaacgga aagagaccct 4020
   ctacgctaca ccagatgaat accaagctga gtgccgtgta tgccggagaa gccatgcaca 4080
   cggccgggtg tgcaccatcc tacagagtta agagagcaga catagccacg tgcacagaag 4140
   cggctgtggt taacgcagct aacgcccgtg gaactgtagg ggatggcgta tgcagggccg 4200
   tggcgaagaa atggccgtca gcctttaagg gagcagcaac accagtgggc acaattaaaa 4260
   cagtcatgtg cggctcgtac cccgtcatcc acgctgtagc gcctaatttc tctgccacga 4320
   ctgaagcgga aggggaccgc gaattggccg ctgtctaccg ggcagtggcc gccgaagtaa 4380
   acagactgtc actgagcagc gtagccatcc cgctgctgtc cacaggagtg ttcagcggcg 4440
   gaagagatag gctgcagcaa tccctcaacc atctattcac agcaatggac gccacggacg 4500
   ctgacgtgac catctactgc agagacaaaa gttgggagaa gaaaatccag gaagccattg 4560
   acatgaggac ggctgtggag ttgctcaatg atgacgtgga gctgaccaca gacttggtga 4620
   gagtgcaccc ggacagcagc ctggtgggtc gtaagggcta cagtaccact gacgggtcgc 4680
   tgtactcgta ctttgaaggt acgaaattca accaggctgc tattgatatg gcagagatac 4740
   tgacgttgtg gcccagactg caagaggcaa acgaacagat atgcctatac gcgctgggcg 4800
   aaacaatgga caacatcaga tccaaatgtc cggtgaacga ttccgattca tcaacacctc 4860
   ccaggacagt gccctgcctg tgccgctacg caatgacagc agaacggatc gcccgcctta 4920
   ggtcacacca agttaaaagc atggtggttt gctcatcttt tcccctcccg aaataccatg 4980
   tagatggggt gcagaaggta aagtgcgaga aggttctcct gttcgacccg acggtacctt 5040
   cagtggttag tccgcggaag tatgccgcat ctacgacgga ccactcagat cggtcgttac 5100
   gagggtttga cttggactgg accaccgact cgtcttccac tgccagcgat accatgtcgc 5160
   tacccagttt gcagtcgtgt gacatcgact cgatctacga gccaatggct cccatagtag 5220
   tgacggctga cgtacaccct gaacccgcag gcatcgcgga cctggcggca gatgtgcacc 5280
   ctgaacccgc agaccatgtg gacctcgaga acccgattcc tccaccgcgc ccgaagagag 5340
   ctgcatacct tgcctcccgc gcggcggagc gaccggtgcc ggcgccgaga aagccgacgc 5400
   ctgccccaag gactgcgttt aggaacaagc tgcctttgac gttcggcgac tttgacgagc 5460
   acgaggtcga tgcgttggcc tccgggatta ctttcggaga cttcgacgac gtcctgcgac 5520
   taggccgcgc gggtgcatat attttctcct cggacactgg cagcggacat ttacaacaaa 5580
   aatccgttag gcagcacaat ctccagtgcg cacaactgga tgcggtccag gaggagaaaa 5640
   tgtacccgcc aaaattggat actgagaggg agaagctgtt gctgctgaaa atgcagatgc 5700
   acccatcgga ggctaataag agtcgatacc agtctcgcaa agtggagaac atgaaagcca 5760
   cggtggtgga caggctcaca tcgggggcca gattgtacac gggagcggac gtaggccgca 5820
   taccaacata cgcggttcgg tacccccgcc ccgtgtactc ccctaccgtg atcgaaagat 5880
   tctcaagccc cgatgtagca atcgcagcgt gcaacgaata cctatccaga aattacccaa 5940
   cagtggcgtc gtaccagata acagatgaat acgacgcata cttggacatg gttgacgggt 6000
   cggatagttg cttggacaga gcgacattct gcccggcgaa gctccggtgc tacccgaaac 6060
   atcatgcgta ccaccagccg actgtacgca gtgccgtccc gtcacccttt cagaacacac 6120
   tacagaacgt gctagcggcc gccaccaaga gaaactgcaa cgtcacgcaa atgcgagaac 6180
   tacccaccat ggactcggca gtgttcaacg tggagtgctt caagcgctat gcctgctccg 6240
   gagaatattg ggaagaatat gctaaacaac ctatccggat aaccactgag aacatcacta 6300
   cctatgtgac caaattgaaa ggcccgaaag ctgctgcctt gttcgctaag acccacaact 6360
   tggttccgct gcaggaggtt cccatggaca gattcacggt cgacatgaaa cgagatgtca 6420
   aagtcactcc agggacgaaa cacacagagg aaagacccaa agtccaggta attcaagcag 6480
   cggagccatt ggcgaccgct tacctgtgcg gcatccacag ggaattagta aggagactaa 6540
   atgctgtgtt acgccctaac gtgcacacat tgtttgatat gtcggccgaa gactttgacg 6600
   cgatcatcgc ctctcacttc cacccaggag acccggttct agagacggac attgcatcat 6660
   tcgacaaaag ccaggacgac tccttggctc ttacaggttt aatgatcctc gaagatctag 6720
   gggtggatca gtacctgctg gacttgatcg aggcagcctt tggggaaata tccagctgtc 6780
   acctaccaac tggcacgcgc ttcaagttcg gagctatgat gaaatcgggc atgtttctga 6840
   ctttgtttat taacactgtt ttgaacatca ccatagcaag cagggtactg gagcagagac 6900
   tcactgactc cgcctgtgcg gccttcatcg gcgacgacaa catcgttcac ggagtgatct 6960
   ccgacaagct gatggcggag aggtgcgcgt cgtgggtcaa catggaggtg aagatcattg 7020
   acgctgtcat gggcgaaaaa cccccatatt tttgtggggg attcatagtt tttgacagcg 7080
   tcacacagac cgcctgccgt gtttcagacc cacttaagcg cctgttcaag ttgggtaagc 7140
   cgctaacagc tgaagacaag caggacgaag acaggcgacg agcactgagt gacgaggtta 7200
   gcaagtggtt ccggacaggc ttgggggccg aactggaggt ggcactaaca tctaggtatg 7260
   aggtagaggg ctgcaaaagt atcctcatag ccatggccac cttggcgagg gacattaagg 7320
   cgtttaagaa attgagagga cctgttatac acctctacgg cggtcctaga ttggtgcgtt 7380
   aatacacaga attctgattg gatcatagcg cactattata ggatccagat cccgggtaat 7440
   taattgaatt acatccctac gcaaacgttt tacggccgcc ggtggcgccc gcgcccggcg 7500
   gcccgtcctt ggccgttgca ggccactccg gtggctcccg tcgtccccga cttccaggcc 7560
   cagcagatgc agcaactcat cagcgccgta aatgcgctga caatgagaca gaacgcaatt 7620
   gctcctgcta ggcctcccaa accaaagaag aagaagacaa ccaaaccaaa gccgaaaacg 7680
   cagcccaaga agatcaacgg aaaaacgcag cagcaaaaga agaaagacaa gcaagccgac 7740
   aagaagaaga agaaacccgg aaaaagagaa agaatgtgca tgaagattga aaatgactgt 7800
   atcttcgtat gcggctagcc acagtaacgt agtgtttcca gacatgtcgg gcaccgcact 7860
   atcatgggtg cagaaaatct cgggtggtct gggggccttc gcaatcggcg ctatcctggt 7920
   gctggttgtg gtcacttgca ttgggctccg cagataagtt agggtaggca atggcattga 7980
   tatagcaaga aaattgaaaa cagaaaaagt tagggtaagc aatggcatat aaccataact 8040
   gtataacttg taacaaagcg caacaagacc tgcgcaattg gccccgtggt ccgcctcacg 8100
   gaaactcggg gcaactcata ttgacacatt aattggcaat aattggaagc ttacataagc 8160
   ttaattcgac gaataattgg atttttattt tattttgcaa ttggttttta atatttccaa 8220
   aaaaaaaaaa aaaaaaaaaa aaaaaaaaaa aaaaaaaaaa aaaaaaaaaa aaaaaaaaaa 8280
   aaaaaaaact agtctgcatt aatgaatcgg ccaacgcgcg gggagaggcg gtttgcgtat 8340
   tgggcgctct tccgcttcct cgctcactga ctcgctgcgc tcggtcgttc ggctgcggcg 8400
   agcggtatca gctcactcaa aggcggtaat acggttatcc acagaatcag gggataacgc 8460
   aggaaagaac atgtgagcaa aaggccagca aaaggccagg aaccgtaaaa aggccgcgtt 8520
   gctggcgttt ttccataggc tccgcccccc tgacgagcat cacaaaaatc gacgctcaag 8580
   tcagaggtgg cgaaacccga caggactata aagataccag gcgtttcccc ctggaagctc 8640
   cctcgtgcgc tctcctgttc cgaccctgcc gcttaccgga tacctgtccg cctttctccc 8700
   ttcgggaagc gtggcgcttt ctcaatgctc gcgctgtagg tatctcagtt cggtgtaggt 8760
   cgttcgctcc aagctgggct gtgtgcacga accccccgtt cagcccgacc gctgcgcctt 8820
   atccggtaac tatcgtcttg agtccaaccc ggtaagacac gacttatcgc cactggcagc 8880
   agccactggt aacaggatta gcagagcgag gtatgtaggc ggtgctacag agttcttgaa 8940
   gtggtggcct aactacggct acactagaag gacagtattt ggtatctgcg ctctgctgaa 9000
   gccagttacc ttcggaaaaa gagttggtag ctcttgatcc ggcaaacaaa ccaccgctgg 9060
   tagcggtggt ttttttgttt gcaagcagca gattacgcgc agaaaaaaag gatctcaaga 9120
   agatcctttg atcttttcta cggggtctga cgctcagtgg aacgaaaact cacgttaagg 9180
   gattttggtc atgagattat caaaaaggat cttcacctag atccttttaa attaaaaatg 9240
   aagttttaaa tcaatctaaa gtatatatga gtaaacttgg tctgacagtt accaatgctt 9300
   aatcagtgag gcacctatct cagcgatctg tctatttcgt tcatccatag ttgcctgact 9360
   ccccgtcgtg tagataacta cgatacggga gggcttacca tctggcccca gtgctgcaat 9420
   gataccgcga gacccacgct caccggctcc agatttatca gcaataaacc agccagccgg 9480
   aagggccgag cgcagaagtg gtcctgcaac tttatccgcc tccatccagt ctattaattg 9540
   ttgccgggaa gctagagtaa gtagttcgcc agttaatagt ttgcgcaacg ttgttgccat 9600
   tgctacaggc atcgtggtgt cacgctcgtc gtttggtatg gcttcattca gctccggttc 9660
   ccaacgatca aggcgagtta catgatcccc catgttgtgc aaaaaagcgg ttagctcctt 9720
   cggtcctccg atcgttgtca gaagtaagtt ggccgcagtg ttatcactca tggttatggc 9780
   agcactgcat aattctctta ctgtcatgcc atccgtaaga tgcttttctg tgactggtga 9840
   gtactcaacc aagtcattct gagaatagtg tatgcggcga ccgagttgct cttgcccggc 9900
   gtcaatacgg gataataccg cgccacatag cagaacttta aaagtgctca tcattggaaa 9960
   acgttcttcg gggcgaaaac tctcaaggat cttaccgctg ttgagatcca gttcgatgta 10020
   acccactcgt gcacccaact gatcttcagc atcttttact ttcaccagcg tttctgggtg 10080
   agcaaaaaca ggaaggcaaa atgccgcaaa aaagggaata agggcgacac ggaaatgttg 10140
   aatactcata ctcttccttt ttcaatatta ttgaagcatt tatcagggtt attgtctcat 10200
   gagcggatac atatttgaat gtatttagaa aaataaacaa ataggggttc cgcgcacatt 10260
   tccccgaaaa gtgccacctg acgtctaaga aaccattatt atcatgacat taacctataa 10320
   aaataggcgt atcacgaggc cctttcgtct cgcgcgtttc ggtgatgacg gtgaaaacct 10380
   ctgacacatg cagctcccgg agacggtcac agcttctgtc taagcggatg ccgggagcag 10440
   acaagcccgt cagggcgcgt cagcgggtgt tggcgggtgt cggggctggc ttaactatgc 10500
   ggcatcagag cagattgtac tgagagtgca ccatatcgac gctctccctt atgcgactcc 10560
   tgcattagga agcagcccag tactaggttg aggccgttga gcaccgccgc cgcaaggaat 10620
   ggtgcatgca aggagatggc gcccaacagt cccccggcca cggggcctgc caccataccc 10680
   acgccgaaac aagcgctcat gagcccgaag tggcgagccc gatcttcccc atcggtgatg 10740
   tcggcgatat aggcgccagc aaccgcacct gtggcgccgg tgatgccggc cacgatgcgt 10800
   ccggcgtaga ggatctggct agcgatgacc ctgctgattg gttcgctgac catttccggg 10860
   gtgcggaacg gcgttaccag aaactcagaa ggttcgtcca accaaaccga ctctgacggc 10920
   agtttacgag agagatgata gggtctgctt cagtaagcca gatgctacac aattaggctt 10980
   gtacatattg tcgttagaac gcggctacaa ttaatacata accttatgta tcatacacat 11040
   acgatttagg tgacactata 11060
<210> 5
   <211> 27
   <212> DNA
   <213> Semliki Forest virus
<220>
<400> 5
   gatcatagcg cactattata ggatcca 27
<210> 6
   <211> 27
   <212> DNA
   <213> SemliKi Forest virus
<220>
<400> 6
   tatcgcgtga raatalcota ggtctag 27
<210> 7
   <211> 31
   <212> DNA
   <213> Semliki Forest virus
<220>
<400> 7
   aacccacgac atcatactag Latatagatc t 31
<210> 8
   <211> 31
   <212> DNA
   <213> Semliki Forest virus
<220>
<400> 8
   gtactatagt argatcatat atctagdtca a 31
<210> 9
   <211> 86
   <212> DNA
   <213> Semliki Forest virus
<220>
<400> 9
   cgggtcggca tggcatctcc acctcctcgc ggtccgacct gggcatccga aggaggacgc 60
   acgtccactc ggatggctaa gggaga                                      86
<210> 10
   <211> 90
   <212> DNA
   <213> Semliki Forest virus
<220>
<400> 10
   gcccagccgt accgtagagg tggaggagcg ccaggctgga cccgtaggct tcctcctgcg 60
   tgcaggtgag cctaccgatt ccctctctag                                  90
<210> 11
   <211> 12474
   <212> DNA
   <213> Semliki Forest virus
<220>
<400> 11
   attggctatt ggccattgca tacgttgtat ctatatcata atatgtacat ttatattggc 60
   tcatgtccaa tatgaccgcc atgttgacat tgattattga ctagttatta atagtaatca 120
   attacggggt cattagttca tagcccatat atggagttcc gcgttacata acttacggta 180
   aatggcccgc ctcgtgaccg cccaacgacc cccgcccatt gacgtcaata atgacgtatg 240
   ttcccatagt aacgccaata gggactttcc attgacgtca atgggtggag tatttacggt 300
   aaactgccca cttggcagta catcaagtgt atcatatgcc aagtccgccc cctattgacg 360
   tcaatgacgg taaatggccc gcctggcatt atgcccagta catgacctta cgggactttc 420
   ctacttggca gtacatctac gtattagtca tcgctattac catggtgatg cggttttggc 480
   agtacaccaa tgggcgtgga tagcggtttg actcacgggg atttccaagt ctccacccca 540
   ttgacgtcaa tgggagtttg ttttggcacc aaaatcaacg ggactttcca aaatgtcgta 600
   ataaccccgc cccgttgacg caaatgggcg gtaggcgtgt acggtgggag gtctatataa 660
   gcagagctcg tttagtgaac cgtatggcgg atgtgtgaca tacacgacgc caaaagattt 720
   tgttccagct cctgccacct ccgctacgcg agagattaac cacccacgat ggccgccaaa 780
   gtgcatgttg atattgaggc tgacagccca ttcatcaagt ctttgcagaa ggcatttccg 840
   tcgttcgagg tggagtcatt gcaggtcaca ccaaatgacc atgcaaatgc cagagcattt 900
   tcgcacctgg ctaccaaatt gatcgagcag gagactgaca aagacacact catcttggat 960
   atcggcagtg cgccttccag gagaatgatg tctacgcaca aataccactg cgtatgccct 1020
   atgcgcagcg cagaagaccc cgaaaggctc gatagctacg caaagaaact ggcagcggcc 1080
   tccgggaagg tgctggatag agagatcgca ggaaaaatca ccgacctgca gaccgtcatg 1140
   gctacgccag acgctgaatc tcctaccttt tgcctgcata cagacgtcac gtgtcgtacg 1200
   gcagccgaag tggccgtata ccaggacgtg tatgctgtac atgcaccaac atcgctgtac 1260
   catcaggcga tgaaaggtgt cagaacggcg tattggattg ggtttgacac caccccgttt 1320
   atgtttgacg cgctagcagg cgcgtatcca acctacgcca caaactgggc cgacgagcag 1380
   gtgttacagg ccaggaacat aggactgtgt gcagcatcct tgactgaggg aagactcggc 1440
   aaactgtcca ttctccgcaa gaagcaattg aaaccttgcg acacagtcat gttctcggta 1500
   ggatctacat tgtacactga gagcagaaag ctactgagga gctggcactt accctccgta 1560
   ttccacctga aaggtaaaca atcctttacc tgtaggtgcg ataccatcgt atcatgtgaa 1620
   gggtacgtag ttaagaaaat cactatgtgc cccggcctgt acggtaaaac ggtagggtac 1680
   gccgtgacgt atcacgcgga gggattccta gtgtgcaaga ccacagacac tgtcaaagga 1740
   gaaagagtct cattccctgt atgcacctac gtcccctcaa ccatctgtga tcaaatgact 1800
   ggcatactag cgaccgacgt cacaccggag gacgcacaga agttgttagt gggattgaat 1860
   cagaggatag ttgtgaacgg aagaacacag cgaaacacta acacgatgaa gaactatctg 1920
   cttccgattg tggccgtcgc atttagcaag tgggcgaggg aatacaaggc agaccttgat 1980
   gatgaaaaac ctctgggtgt ccgagagagg tcacttactt gctgctgctt gtgggcattt 2040
   aaaacgagga agatgcacac catgtacaag aaaccagaca cccagacaat agtgaaggtg 2100
   ccttcagagt ttaactcgtt cgtcatcccg agcctatggt ctacaggcct cgcaatccca 2160
   gtcagatcac gcattaagat gcttttggcc aagaagacca agcgagagtt aatacctgtt 2220
   ctcgacgcgt cgtcagccag ggatgctgaa caagaggaga aggagaggtt ggaggccgag 2280
   ctgactagag aagccttacc acccctcgtc cccatcgcgc cggcggagac gggagtcgtc 2340
   gacgtcgacg ttgaagaact agagtatcac gcaggtgcag ggtcgtggaa aacacctcgc 2400
   agcgcgttga aagtcaccgc acagccgaac gacgtactac taggaaatta cgtagttctg 2460
   tccccgcaga ccgtgctcaa gagctccaag ttggcccccg tgcaccctct agcagagcag 2520
   gtgaaaataa taacacataa cgggagggcc ggcggttacc aggtcgacgg atatgacggc 2580
   agggtcctac taccatgtgg atcggccatt ccggtccctg agtttcaagc tttgagcgag 2640
   agcgccacta tggtgtacaa cgaaagggag ttcgtcaaca ggaaactata ccatattgcc 2700
   gttcacggac cgtcgctgaa caccgacgag gagaactacg agaaagtcag agctgaaaga 2760
   actgacgccg agtacgtgtt cgacgtagat aaaaaatgct gcgtcaagag agaggaagcg 2820
   tcgggtttgg tgttggtggg agagctaacc aaccccccgt tccatgaatt cgcctacgaa 2880
   gggctgaaga tcaggccgtc ggcaccatat aagactacag tagtaggagt ctttggggtt 2940
   ccgggatcag gcaagtctgc tattattaag agcctcgtga ccaaacacga tctggtcacc 3000
   agcggcaaga aggagaactg ccaggaaata gttaacgacg tgaagaagca ccgcgggaag 3060
   gggacaagta gggaaaacag tgactccatc ctgctaaacg ggtgtcgtcg tgccgtggac 3120
   atcctatatg tggacgaggc tttcgcttgc cattccggta ctctgctagc cctaattgct 3180
   cttgttaaac ctcggagcaa agtggtgtta tgcggagacc ccaagcaatg cggattcttc 3240
   aatatgatgc agcttaaggt gaacttcaac cacaacatct gcactgaagt atgtcataaa 3300
   agtatatcca gacgttgcac gcgtccagtc acggccatcg tgtctacgtt gcactacgga 3360
   ggcaagatgc gcacgaccaa cccgtgcaac aaacccataa tcatagacac cacaggacag 3420
   accaagccca agccaggaga catcgtgtta acatgcttcc gaggctgggc aaagcagctg 3480
   cagttggact accgtggaca cgaagtcatg acagcagcag catctcaggg cctcacccgc 3540
   aaaggggtat acgccgtaag gcagaaggtg aatgaaaatc ccttgtatgc ccctgcgtcg 3600
   gagcacgtga atgtactgct gacgcgcact gaggataggc tggtgtggaa aacgctggcc 3660
   ggcgatccct ggattaaggt gagtttgggg acccttgatt gttctttctt tttcgctatt 3720
   gtaaaattca tgttatatgg agggggcaaa gttttcaggg tgttgtttag aatgggaaga 3780
   tgtcccttgt atcaccatgg accctcatga taattttgtt tctttcactt tctactctgt 3840
   tgacaaccat tgtctcctct tattttcttt tcattttctg taactttttc gttaaacttt 3900
   agcttgcatt tgtaacgaat ttttaaattc acttttgttt atttgtcaga ttgtaagtac 3960
   tttctctaat cacttttttt tcaaggcaat cagggtatat tatattgtac ttcagcacag 4020
   ttttagagaa caattgttat aattaaatga taaggtagaa tatttctgca tataaattct 4080
   ggctggcgtg gaaatattct tattggtaga aacaactaca tcctggtcat catcctgcct 4140
   ttctctttat ggttacaatg atatacactg tttgagatga ggataaaata ctctgagtcc 4200
   aaaccgggcc cctctgctaa ccatgttcat gccttcttct ttttcctaca ggtcctatca 4260
   aacattccac agggtaactt tacggccaca ttggaagaat ggcaagaaga acacgacaaa 4320
   ataatgaagg tgattgaagg accggctgcg cctgtggacg cgttccagaa caaagcgaac 4380
   gtgtgttggg cgaaaagcct ggtgcctgtc ctggacactg ccggaatcag attgacagca 4440
   gaggagtgga gcaccataat tacagcattt aaggaggaca gagcttactc tccagtggtg 4500
   gccttgaatg aaatttgcac caagtactat ggagttgacc tggacagtgg cctgttttct 4560
   gccccgaagg tgtccctgta ttacgagaac aaccactggg ataacagacc tggtggaagg 4620
   atgtatggat tcaatgccgc aacagctgcc aggctggaag ctagacatac cttcctgaag 4680
   gggcagtggc atacgggcaa gcaggcagtt atcgcagaaa gaaaaatcca accgctttct 4740
   gtgctggaca atgtaattcc tatcaaccgc aggctgccgc acgccctggt ggctgagtac 4800
   aagacggtta aaggcagtag ggttgagtgg ctggtcaata aagtaagagg gtaccacgtc 4860
   ctgctggtga gtgagtacaa cctggctttg cctcgacgca gggtcacttg gttgtcaccg 4920
   ctgaatgtca caggcgccga taggtgctac gacctaagtt taggactgcc ggctgacgcc 4980
   ggcaggttcg acttggtctt tgtgaacatt cacacggaat tcagaatcca ccactaccag 5040
   cagtgtgtcg accacgccat gaagctgcag atgcttgggg gagatgcgct acgactgcta 5100
   aaacccggcg gcatcttgat gagagcttac ggatacgccg ataaaatcag cgaagccgtt 5160
   gtttcctcct taagcagaaa gttctcgtct gcaagagtgt tgcgcccgga ttgtgtcacc 5220
   agcaatacag aagtgttctt gctgttctcc aactttgaca acggaaagag accctctacg 5280
   ctacaccaga tgaataccaa gctgagtgcc gtgtatgccg gagaagccat gcacacggcc 5340
   gggtgtgcac catcctacag agttaagaga gcagacatag ccacgtgcac agaagcggct 5400
   gtggttaacg cagctaacgc ccgtggaact gtaggggatg gcgtatgcag ggccgtggcg 5460
   aagaaatggc cgtcagcctt taagggagca gcaacaccag tgggcacaat taaaacagtc 5520
   atgtgcggct cgtaccccgt catccacgct gtagcgccta atttctctgc cacgactgaa 5580
   gcggaagggg accgcgaatt ggccgctgtc taccgggcag tggccgccga agtaaacaga 5640
   ctgtcactga gcagcgtagc catcccgctg ctgtccacag gagtgttcag cggcggaaga 5700
   gataggctgc agcaatccct caaccatcta ttcacagcaa tggacgccac ggacgctgac 5760
   gtgaccatct actgcagaga caaaagttgg gagaagaaaa tccaggaagc cattgacatg 5820
   aggacggctg tggagttgct caatgatgac gtggagctga ccacagactt ggtgagagtg 5880
   cacccggaca gcagcctggt gggtcgtaag ggctacagta ccactgacgg gtcgctgtac 5940
   tcgtactttg aaggtacgaa attcaaccag gctgctattg atatggcaga gatactgacg 6000
   ttgtggccca gactgcaaga ggcaaacgaa cagatatgcc tatacgcgct gggcgaaaca 6060
   atggacaaca tcagatccaa atgtccggtg aacgattccg attcatcaac acctcccagg 6120
   acagtgccct gcctgtgccg ctacgcaatg acagcagaac ggatcgcccg ccttaggtca 6180
   caccaagtta aaagcatggt ggtttgctca tcttttcccc tcccgaaata ccatgtagat 6240
   ggggtgcaga aggtaaagtg cgagaaggtt ctcctgttcg acccgacggt accttcagtg 6300
   gttagtccgc ggaagtatgc cgcatctacg acggaccact cagatcggtc gttacgaggg 6360
   tttgacttgg actggaccac cgactcgtct tccactgcca gcgataccat gtcgctaccc 6420
   agtttgcagt cgtgtgacat cgactcgatc tacgagccaa tggctcccat agtagtgacg 6480
   gctgacgtac accctgaacc cgcaggcatc gcggacctgg cggcagatgt gcaccctgaa 6540
   cccgcagacc atgtggacct cgagaacccg attcctccac cgcgcccgaa gagagctgca 6600
   taccttgcct cccgcgcggc ggagcgaccg gtgccggcgc cgagaaagcc gacgcctgcc 6660
   ccaaggactg cgtttaggaa caagctgcct ttgacgttcg gcgactttga cgagcacgag 6720
   gtcgatgcgt tggcctccgg gattactttc ggagacttcg acgacgtcct gcgactaggc 6780
   cgcgcgggtg catatatttt ctcctcggac actggcagcg gacatttaca acaaaaatcc 6840
   gttaggcagc acaatctcca gtgcgcacaa ctggatgcgg tccaggagga gaaaatgtac 6900
   ccgccaaaat tggatactga gagggagaag ctgttgctgc tgaaaatgca gatgcaccca 6960
   tcggaggcta ataagagtcg ataccagtct cgcaaagtgg agaacatgaa agccacggtg 7020
   gtggacaggc tcacatcggg ggccagattg tacacgggag cggacgtagg ccgcatacca 7080
   acatacgcgg ttcggtaccc ccgccccgtg tactccccta ccgtgatcga aagattctca 7140
   agccccgatg tagcaatcgc agcgtgcaac gaatacctat ccagaaatta cccaacagtg 7200
   gcgtcgtacc agataacaga tgaatacgac gcatacttgg acatggttga cgggtcggat 7260
   agttgcttgg acagagcgac attctgcccg gcgaagctcc ggtgctaccc gaaacatcat 7320
   gcgtaccacc agccgactgt acgcagtgcc gtcccgtcac cctttcagaa cacactacag 7380
   aacgtgctag cggccgccac caagagaaac tgcaacgtca cgcaaatgcg agaactaccc 7440
   accatggact cggcagtgtt caacgtggag tgcttcaagc gctatgcctg ctccggagaa 7500
   tattgggaag aatatgctaa acaacctatc cggataacca ctgagaacat cactacctat 7560
   gtgaccaaat tgaaaggccc gaaagctgct gccttgttcg ctaagaccca caacttggtt 7620
   ccgctgcagg aggttcccat ggacagattc acggtcgaca tgaaacgaga tgtcaaagtc 7680
   actccaggga cgaaacacac agaggaaaga cccaaagtcc aggtaattca agcagcggag 7740
   ccattggcga ccgcttacct gtgcggcatc cacagggaat tagtaaggag actaaatgct 7800
   gtgttacgcc ctaacgtgca cacattgttt gatatgtcgg ccgaagactt tgacgcgatc 7860
   atcgcctctc acttccaccc aggagacccg gttctagaga cggacattgc atcattcgac 7920
   aaaagccagg acgactcctt ggctcttaca ggtttaatga tcctcgaaga tctaggggtg 7980
   gatcagtacc tgctggactt gatcgaggca gcctttgggg aaatatccag ctgtcaccta 8040
   ccaactggca cgcgcttcaa gttcggagct atgatgaaat cgggcatgtt tctgactttg 8100
   tttattaaca ctgttttgaa catcaccata gcaagcaggg tactggagca gagactcact 8160
   gactccgcct gtgcggcctt catcggcgac gacaacatcg ttcacggagt gatctccgac 8220
   aagctgatgg cggagaggtg cgcgtcgtgg gtcaacatgg aggtgaagat cattgacgct 8280
   gtcatgggcg aaaaaccccc atatttttgt gggggattca tagtttttga cagcgtcaca 8340
   cagaccgcct gccgtgtttc agacccactt aagcgcctgt tcaagttggg taagccgcta 8400
   acagctgaag acaagcagga cgaagacagg cgacgagcac tgagtgacga ggttagcaag 8460
   tggttccgga caggcttggg ggccgaactg gaggtggcac taacatctag gtatgaggta 8520
   gagggctgca aaagtatcct catagccatg gccaccttgg cgagggacat taaggcgttt 8580
   aagaaattga gaggacctgt tatacacctc tacggcggtc ctagattggt gcgttaatac 8640
   acagaattct gattggatca tagcgcacta ttataggatc cagatcccgg gtaattaatt 8700
   gaattacatc cctacgcaaa cgttttacgg ccgccggtgg cgcccgcgcc cggcggcccg 8760
   tccttggccg ttgcaggcca ctccggtggc tcccgtcgtc cccgacttcc aggcccagca 8820
   gatgcagcaa ctcatcagcg ccgtaaatgc gctgacaatg agacagaacg caattgctcc 8880
   tgctaggcct cccaaaccaa agaagaagaa gacaaccaaa ccaaagccga aaacgcagcc 8940
   caagaagatc aacggaaaaa cgcagcagca aaagaagaaa gacaagcaag ccgacaagaa 9000
   gaagaagaaa cccggaaaaa gagaaagaat gtgcatgaag attgaaaatg actgtatctt 9060
   cgtatgcggc tagccacagt aacgtagtgt ttccagacat gtcgggcacc gcactatcat 9120
   gggtgcagaa aatctcgggt ggtctggggg ccttcgcaat cggcgctatc ctggtgctgg 9180
   ttgtggtcac ttgcattggg ctccgcagat aagttagggt aggcaatggc attgatatag 9240
   caagaaaatt gaaaacagaa aaagttaggg taagcaatgg catataacca taactgtata 9300
   acttgtaaca aagcgcaaca agacctgcgc aattggcccc gtggtccgcc tcacggaaac 9360
   tcggggcaac tcatattgac acattaattg gcaataattg gaagcttaca taagcttaat 9420
   tcgacgaata attggatttt tattttattt tgcaattggt ttttaatatt tccaaaaaaa 9480
   aaaaaaaaaa aaaaaaaaaa aaaaaaaaaa aaaaaaaaaa aaaaaaaaaa aaaaaaaaaa 9540
   aaacgggtcg gcatggcatc tccacctcct cgcggtccga cctgggcatc cgaaggagga 9600
   cgcacgtcca ctcggatggc taagggagat cctgaactta acgctcgagt gccagccatc 9660
   tgttgtttgc ccctcccccg tgccttcctt gaccctggaa ggtgccactc ccactgtcct 9720
   ttcctaataa aatgaggaaa ttgcatcgca ttgtctgagt aggtgtcatt ctattctggg 9780
   gggtggggtg gggcaggaca gcaaggggga ggattgggaa gacaatagca ggcatgctgg 9840
   ggatgcggtg ggctctagga tctcgaccat gcagggtaag gatactgccc ggaacaaaac 9900
   catgatcctg acgccatgcc agcctagttc taggtggagc tccagctttt gttcccttta 9960
   gtgagggtta atttcgagct tggcgtaatc atggtcatag ctgtttcctg tgtgaaattg 10020
   ttatccgctc acaattccac acaacatacg agccggaagc ataaagtgta aagcctgggg 10080
   tgcctaatga gtgagctaac tcacattaat tgcgttgcgc tcactgcccg ctttccagtc 10140
   gggaaacctg tcgtgccagc tgcattaatg aatcggccaa cgcgcgggga gaggcggttt 10200
   gcgtattggg cgctcttccg cttcctcgct cactgactcg ctgcgctcgg tcgttcggct 10260
   gcggcgagcg gtatcagctc actcaaaggc ggtaatacgg ttatccacag aatcagggga 10320
   taacgcagga aagaacatgt gagcaaaagg ccagcaaaag gccaggaacc gtaaaaaggc 10380
   cgcgttgctg gcgtttttcc ataggctccg cccccctgac gagcatcaca aaaatcgacg 10440
   ctcaagtcag aggtggcgaa acccgacagg actataaaga taccaggcgt ttccccctgg 10500
   aagctccctc gtgcgctctc ctgttccgac cctgccgctt accggatacc tgtccgcctt 10560
   tctcccttcg ggaagcgtgg cgctttctca tagctcacgc tgtaggtatc tcagttcggt 10620
   gtaggtcgtt cgctccaagc tgggctgtgt gcacgaaccc cccgttcagc ccgaccgctg 10680
   cgccttatcc ggtaactatc gtcttgagtc caacccggta agacacgact tatcgccact 10740
   ggcagcagcc actggtaaca ggattagcag agcgaggtat gtaggcggtg ctacagagtt 10800
   cttgaagtgg tggcctaact acggctacac tagaaggaca gtatttggta tctgcgctct 10860
   gctgaagcca gttaccttcg gaaaaagagt tggtagctct tgatccggca aacaaaccac 10920
   cgctggtagc ggtggttttt ttgtttgcaa gcagcagatt acgcgcagaa aaaaaggatc 10980
   tcaagaagat cctttgatct tttctacggg gtctgacgct cagtggaacg aaaactcacg 11040
   ttaagggatt ttggtcatga gattatcaaa aaggatcttc acctagatcc ttttaaatta 11100
   aaaatgaagt tttaaatcaa tctaaagtat atatgagtaa acttggtctg acagttacca 11160
   atgcttaatc agtgaggcac ctatctcagc gatctgtcta tttcgttcat ccatagttgc 11220
   ctgactccgg gggggggggg cgctgaggtc tgcctcgtga agaaggtgtt gctgactcat 11280
   accaggcctg aatcgcccca tcatccagcc agaaagtgag ggagccacgg ttgatgagag 11340
   ctttgttgta ggtggaccag ttggtgattt tgaacttttg ctttgccacg gaacggtctg 11400
   cgttgtcggg aagatgcgtg atctgatcct tcaactcagc aaaagttcga tttattcaac 11460
   aaagccgccg tcccgtcaag tcagcgtaat gctctgccag tgttacaacc aattaaccaa 11520
   ttctgattag aaaaactcat cgagcatcaa atgaaactgc aatttattca tatcaggatt 11580
   atcaatacca tatttttgaa aaagccgttt ctgtaatgaa ggagaaaact caccgaggca 11640
   gttccatagg atggcaagat cctggtatcg gtctgcgatt ccgactcgtc caacatcaat 11700
   acaacctatt aatttcccct cgtcaaaaat aaggttatca agtgagaaat caccatgagt 11760
   gacgactgaa tccggtgaga atggcaaaag cttatgcatt tctttccaga cttgttcaac 11820
   aggccagcca ttacgctcgt catcaaaatc actcgcatca accaaaccgt tattcattcg 11880
   tgattgcgcc tgagcgagac gaaatacgcg atcgctgtta aaaggacaat tacaaacagg 11940
   aatcgaatgc aaccggcgca ggaacactgc cagcgcatca acaatatttt cacctgaatc 12000
   aggatattct tctaatacct ggaatgctgt tttcccgggg atcgcagtgg tgagtaacca 12060
   tgcatcatca ggagtacgga taaaatgctt gatggtcgga agaggcataa attccgtcag 12120
   ccagtttagt ctgaccatct catctgtaac atcattggca acgctacctt tgccatgttt 12180
   cagaaacaac tctggcgcat cgggcttccc atacaatcga tagattgtcg cacctgattg 12240
   cccgacatta tcgcgagccc atttataccc atataaatca gcatccatgt tggaatttaa 12300
   tcgcggcctc gagcaagacg tttcccgttg aatatggctc ataacacccc ttgtattact 12360
   gtttatgtaa gcagacagtt ttattgttca tgatgatata tttttatctt gtgcaatgta 12420
   acatcagaga ttttgagaca caacgtggct ttcccccccc cccccgagct tgat       12474
<210> 12
   <211> 292
   <212> DNA
   <213> Semliki Forest virus
<220>
<400> 12
   cgtttagtga accgtatggc ggatgtgtga catacacgac gccaaaagat tttgttccag 60
   ctcctgccac ctccgctacg cgagagatta accacccacg atggccgcca aagtgcatgt 120
   tgatattgag gctgacagcc cattcatcaa gtctttgcag aaggcatttc cgtcgttcga 180
   ggtggagtca ttgcaggtca caccaaatga ccatgcaaat gccagagcat tttcgcacct 240
   ggctaccaaa ttgatcgagc aggagactga caaagacaca ctcatcttgg at         292

## Description

### FIELD OF INVENTION

The present invention relates to the field of DNA vaccines and is particularly concerned with modified alpha virus vectors for use in such vaccines.

### BACKGROUND OF THE INVENTION

Semliki Forest virus (SFV) is a member of the Alphavirus genus in the Togaviridae family. The mature virus particle contains a single copy of a ssRNA genome with a positive polarity that is 5'-capped and 3'-polyadenylated. It functions as an mRNA and naked RNA can start an infection when introduced into cells. Upon infection/transfection, the 5' two-thirds of the genome is translated into a polyprotein that is processed into the four nonstructural proteins (nsP1 to 4) by self cleavage. Once the ns proteins have been synthesized they are responsible for replicating the plus-strand (42S) genoine into full-length minus strands (ref. 14). These minus-strands then serve as templates for the synthesis of new plus-strand (42S) genomes and the 26S subgenomic mRNA (ref. 1 - Throughout this application various references are cited in parentheses to describe more fully the state of the art to which this invention pertains. Full bibliographic information for each citation is found at the end of the specification. This subgenomic mRNA, which is colinear with the last one-third of the genome, encodes the SFV structural proteins. In 1991 Liljestrom and Garoff (ref. 2) designed a series of expression vectors based on the SFV CDNA replicon. These vectors had the virus structural protein genes deleted to make the way for heterologous inserts, but preserved the nonstructural coding region for production of the nsPl to 4 replicase complex. Short 5' and 3' sequence elements required for RNA replication were also preserved. A polylinker site was inserted downstream from the 26S promoter followed by translation stop sites in all three frames. An SpeI site was inserted just after the 3' end of the SFV CDNA for linearization of the plasmid for use *in vitro* transcription reactions.

Injection of SFV RNA encoding a heterologous protein have been shown to result in the expression of the foreign protein and the induction of antibody in a number of studies (refs. 3,4). The use of SFV RNA inoculation to express foreign proteins for the purpose of immunization would have several of the advantages associated with plasmid DNA immunization. For example, SFV RNA encoding a viral antigen may be introduced in the presence of antibody to that virus without a loss in potency due to neutralization by antibodies to the virus. Also, because the protein is expressed *in vivo* the protein should have the same conformation as the protein expressed by the virus itself. Therefore, concerns about conformational changes which could occur during protein purification leading to a loss in immunogenicity, protective epitopes and possibly immunopotentiation, could be avoided by plasmid DNA immunization.

In WO95/27044, there is described the use of alphavirus cDNA vectors based, on cDNA complementary to the alphavirus RNA sequence. Once transcribed from the cDNA under transcriptional control of a heterologous promoter, the alphavirus RNA is able to self-replicate by means of its own replicase and thereby amplify the copy number of the transcribed recombinant RNA molecules.

WO 96/40945 describes nucleic acid vaccines for immunization against respiratory syncytial virus. Recombinant vector constructs suitable for the expression of viral proteins are described. The constructs may optionally comprise, *inter alia,* an intron. The specification teaches the general insertion of the intron (e.g. the rabbit β-globin intron II) within the vector, for example, between promoter sequences and sequences encoding the viral protein.

In WO 96/17072 there is described recombinant alphavirus expression vector constructs which optionally comprise, *inter alia,* at least one intron. The specification teaches the insertion of the intron(s) within the vector between the Sindbis (viral) and heterologous gene regions.

### SUMMARY OF THE INVENTION

The present invention is concerned with modifications to the alphavirus cDNA vectors described in the aforementioned WO 95/27044 to permit enhanced replication of the alphavirus. In the present invention, a heterologous splice site is introduced into the alphavirus replicon sequence, particularly that of Semliki Forest virus (SFV).

Accordingly, in one aspect, the present invention provides an expression vector comprising a DNA molecule complementary to at least part of an alphavirus RNA genome, which DNA molecule comprises the complement of the complete alphavirus RNA genome regions which are essential for replication of the said alphavirus RNA, and further comprises a heterologous DNA sequence capable of expression in a suitable host, such as a human or animal host, said heterologous DNA sequence being inserted into a region of the DNA molecule which is non-essential to replication thereof, and the DNA molecule being placed under transcriptional control of a promoter sequence functional in said animal or human host, wherein at least one heterologous splice site is provided in the DNA molecule to prevent aberrant RNA splicing of the alphavirus.

The alphavirus molecule is a large molecule and, accordingly, there is a high probability of cryptic splice sites, thereby impairing the replication of the alphavirus and hence its ability to express the heterlogous DNA is impaired. By introducing the at least one optimal heterologous splice site in accordance with the present invention into the alphavirus replicon sequence, any splicing is likely to be directed at the heterologous splice site rather than any cryptic splice sites, restores the function of the SFV replicon when removed, and may improve transport of RNA from the nucleus (ref. 6) .

In the constructs provided herein, the promoter is placed upstream of the 5'-end of the alphavirus sequence, such that the resultant transcript has an authentic 5'-end, which is required for the efficient replication of the alphavirus RNA replicon.

In addition, there may be provided at the 3'end of the Semliki Forest virus segment, a hepatitis delta virus ribozyme sequence to ensure proper *in vivo* cleavage at the 3'-end of the sequence. Any other convenient sequence may be employed to achieve this effect.

The heterologous splice site sequence may be provided by the nucleotide sequence of the rabbit β-globin intron II, as described in reference 5. Such heterologous splice site sequence may be inserted into the complement sequence at any convenient location which generates perfect splice junctions. This non-essential to replication thereof; a promoter sequence functional in said host cell and transcriptionally controlling said DNA molecule, said promoter sequence being placed upstream of the 5'-end of the DNA molecule such that the resultant transcript had an authentic 5' end; at least one heterologous splice site provided in the complement of the DNA molecule to generate perfect splice junctions in the alphavirus in order to prevent aberrant splicing and an additional DNA sequence at the 3'-end of the DNA molecule to direct proper in vivo cleavage at the 3'-end of the reactant mRNA transcript.

### BRIEF DESCRIPTION OF DRAWINGS

Figure 1 shows the DNA sequence of the β-globin introm II including three additional nucleotides at the 3'-end thereof (SEQ ID No:1);

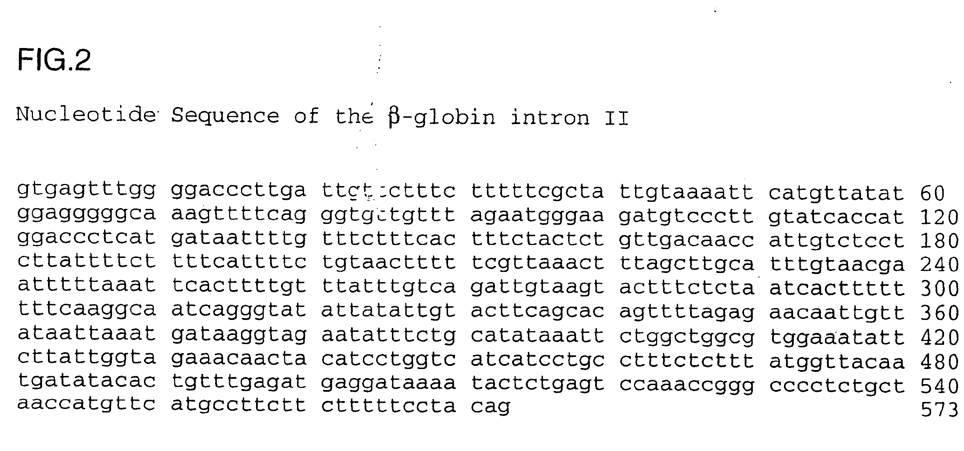

Figure 2 shows the DNA sequence of the β-globin intron II (SEQ ID No:2);

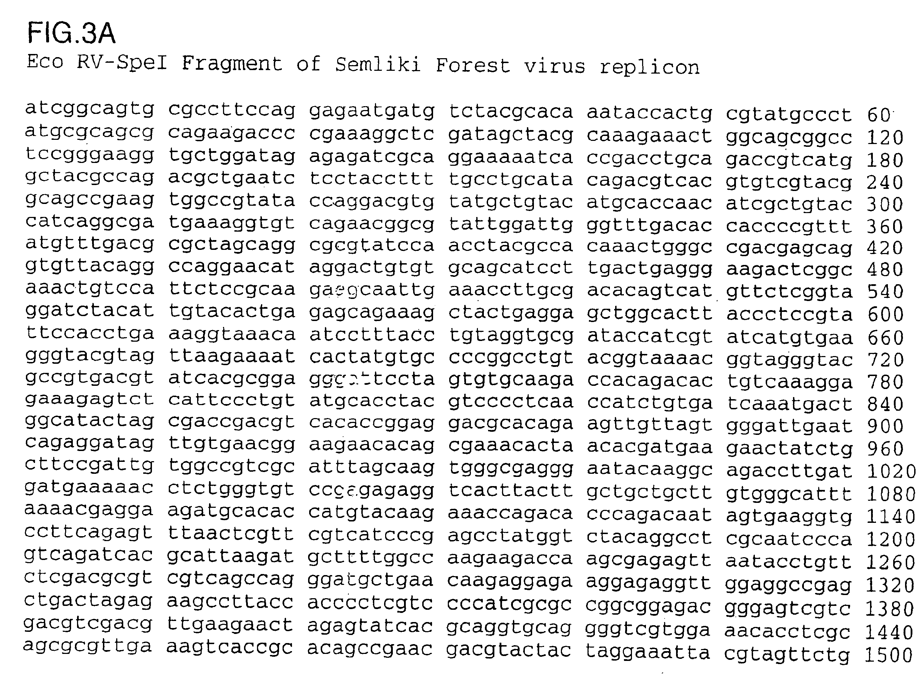

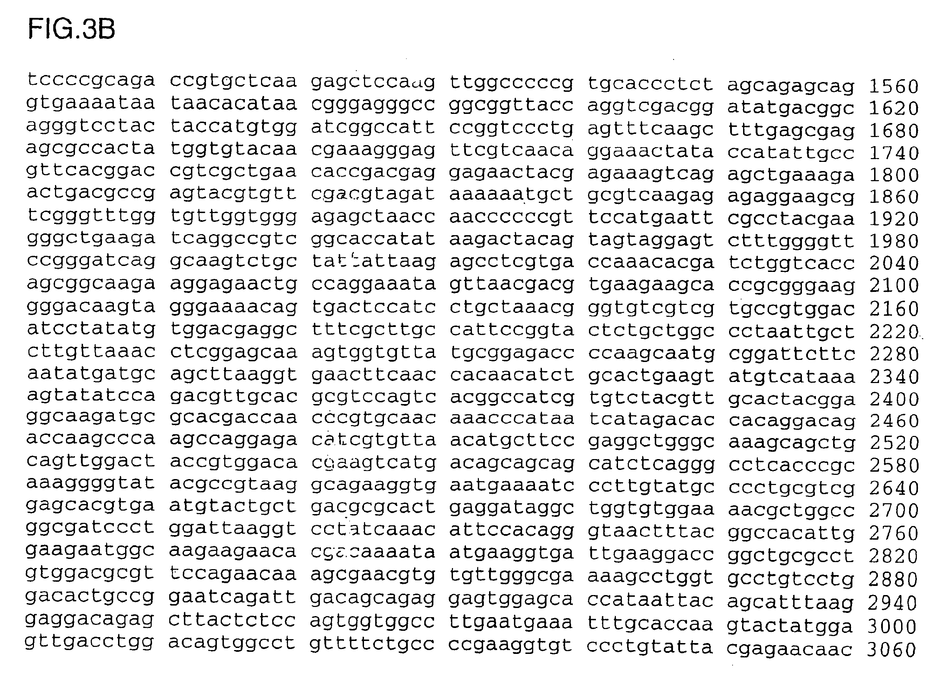

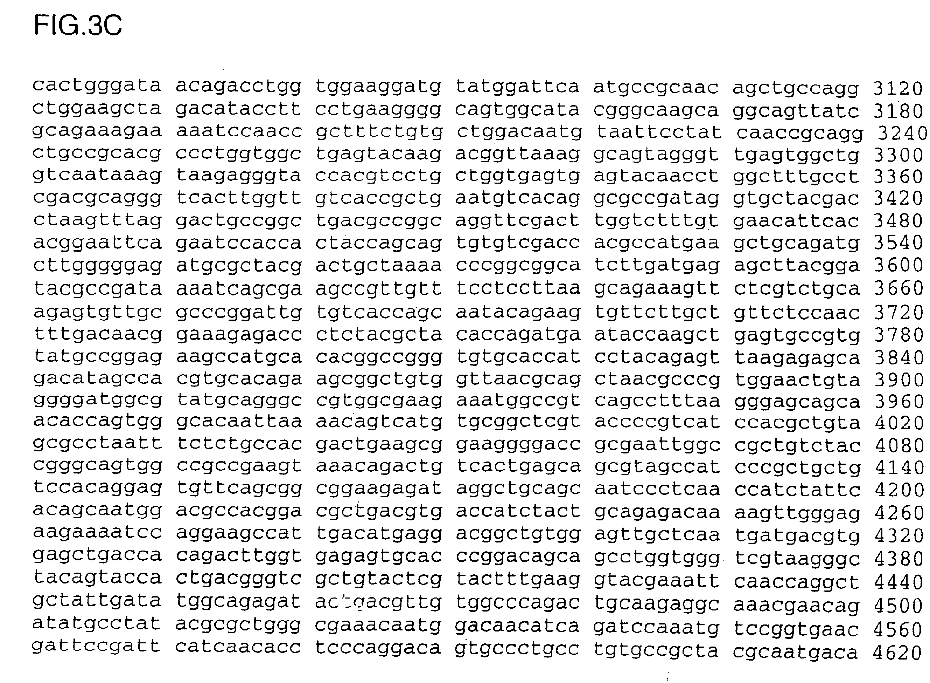

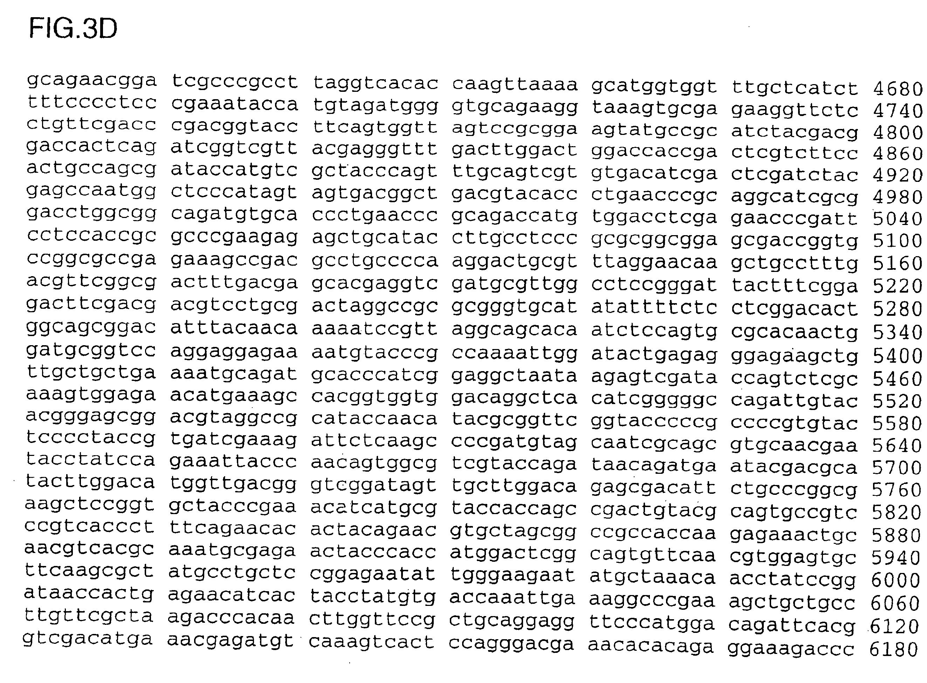

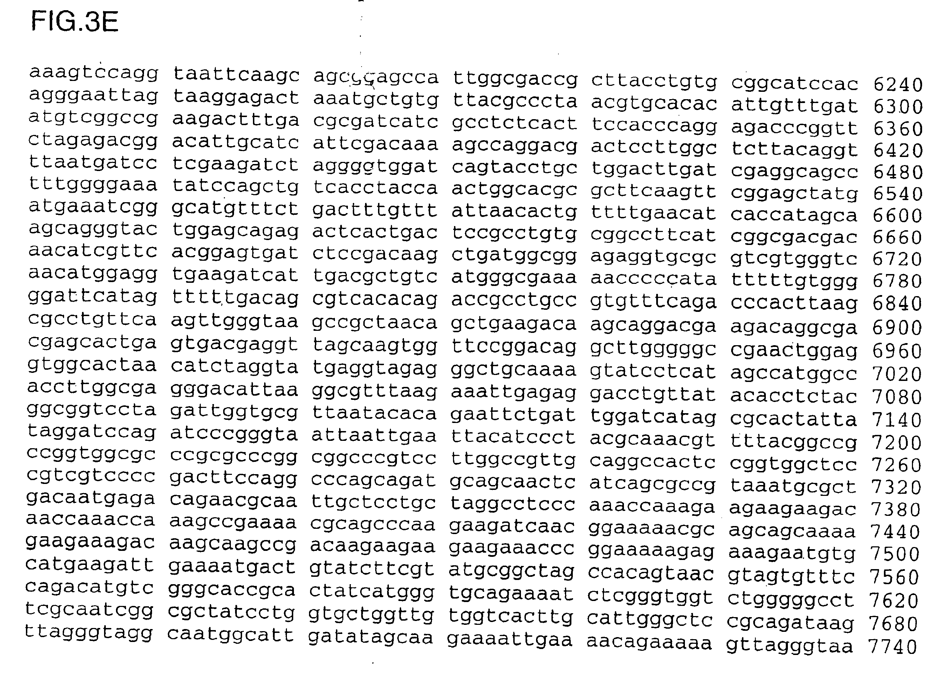

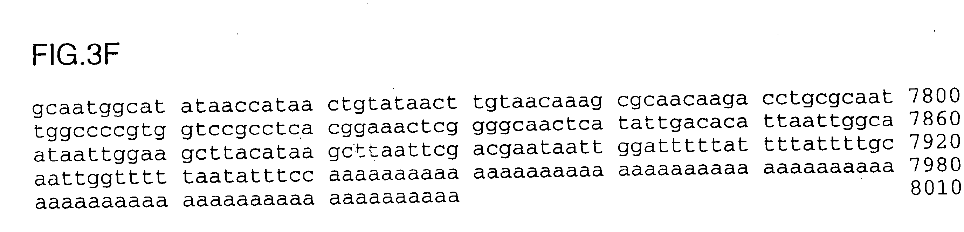

Figures 3A to 3C show the DNA sequence of the EcoRV-Spel fragment of Semliki Forest virus replieon (SEQ ID No:3);
Figures 4A to 4D show the DNA sequence of the pSFV link (SEQ ID no: 4) prepared as illustrated in Figure 5;
Figure 5 shows; construction of pSFVlink (11060 bp) from pSFV1 using a linker sequence (SEQ ID nos: 5, 6);

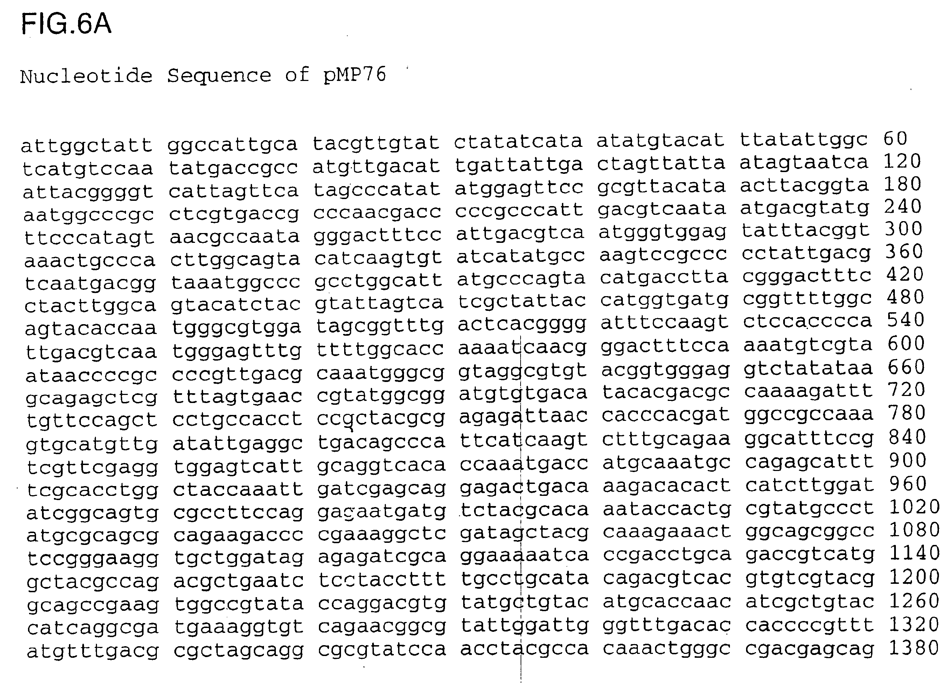

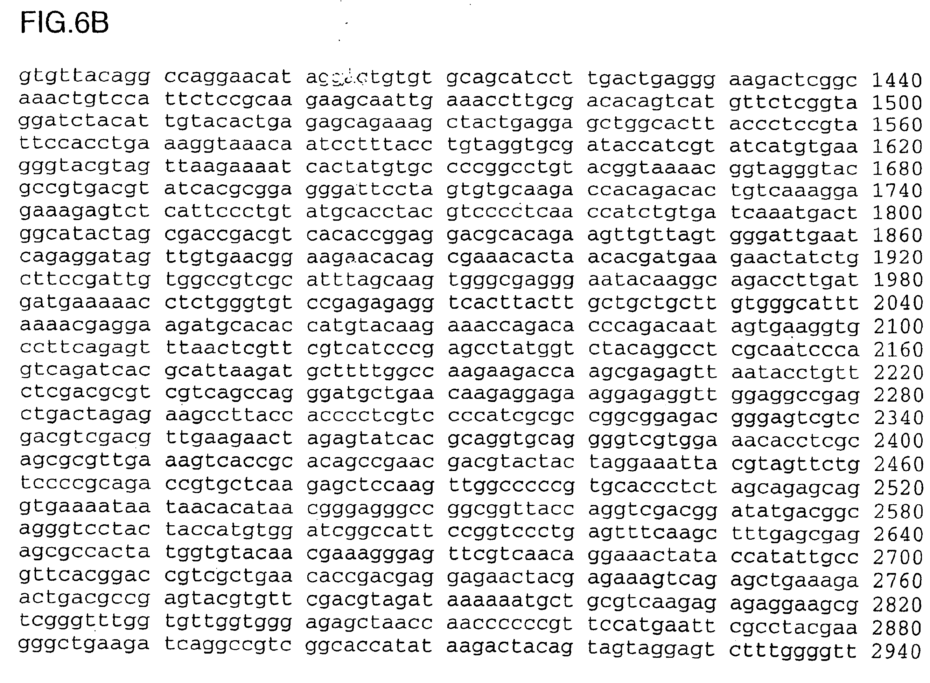

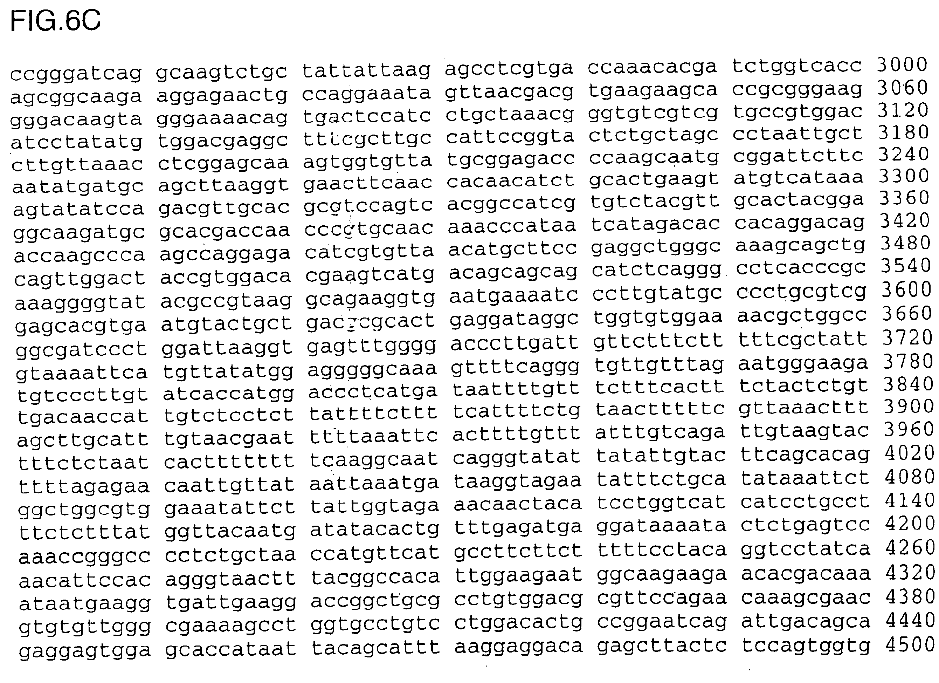

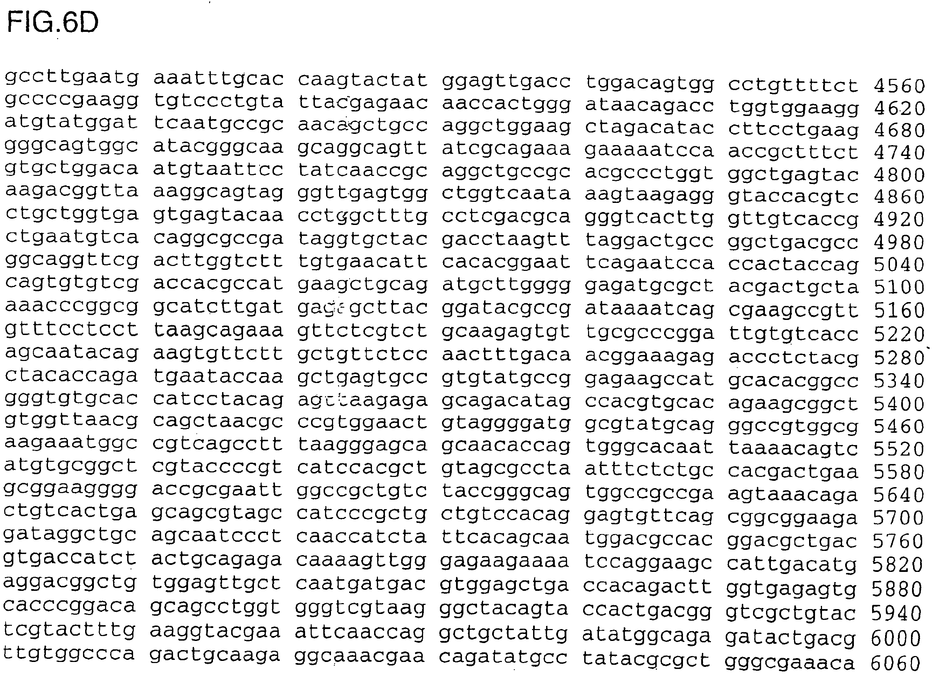

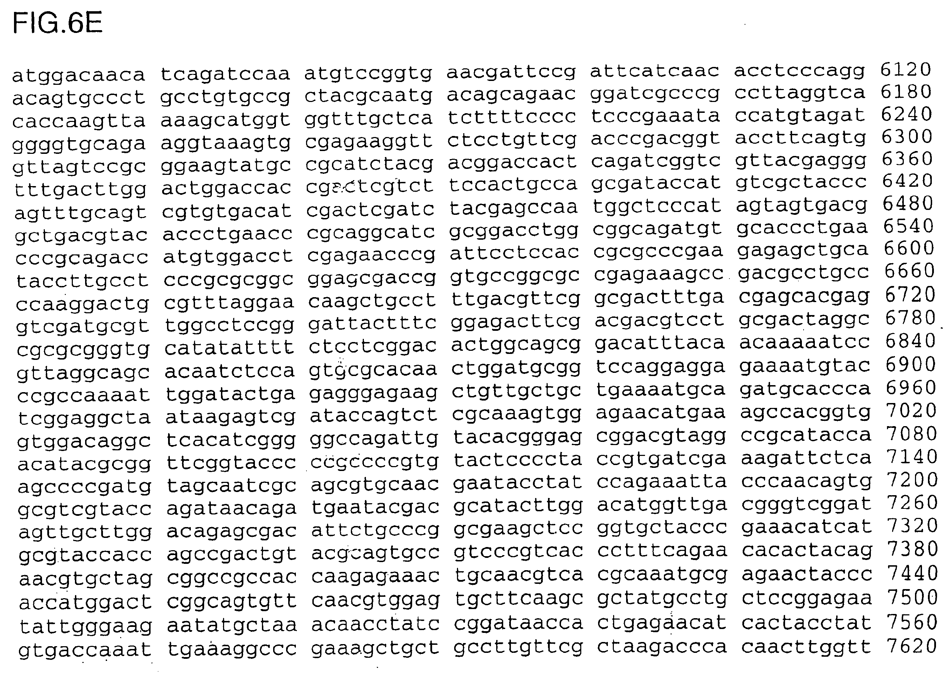

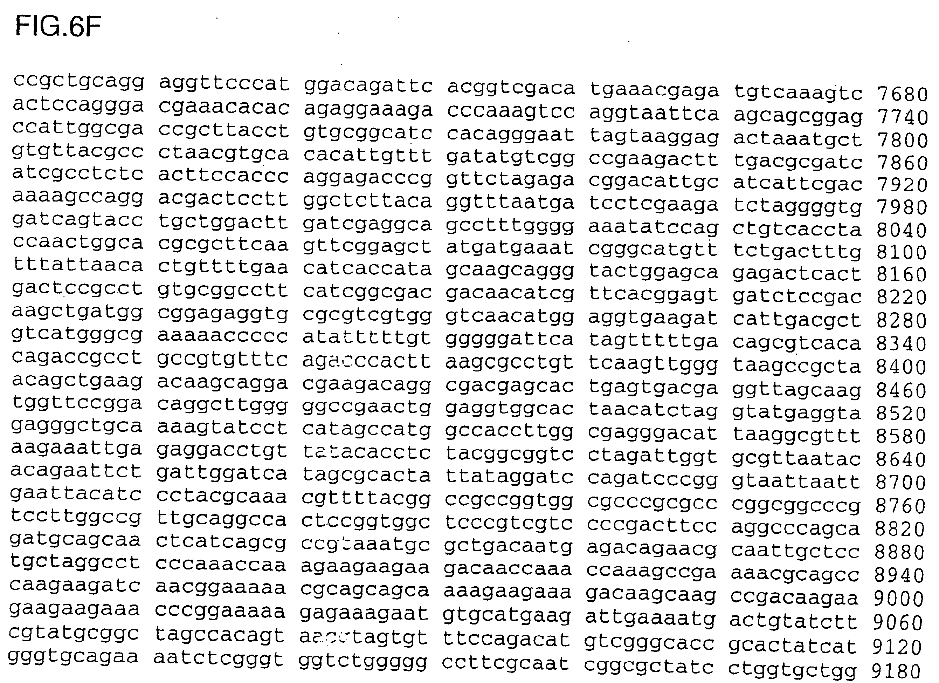

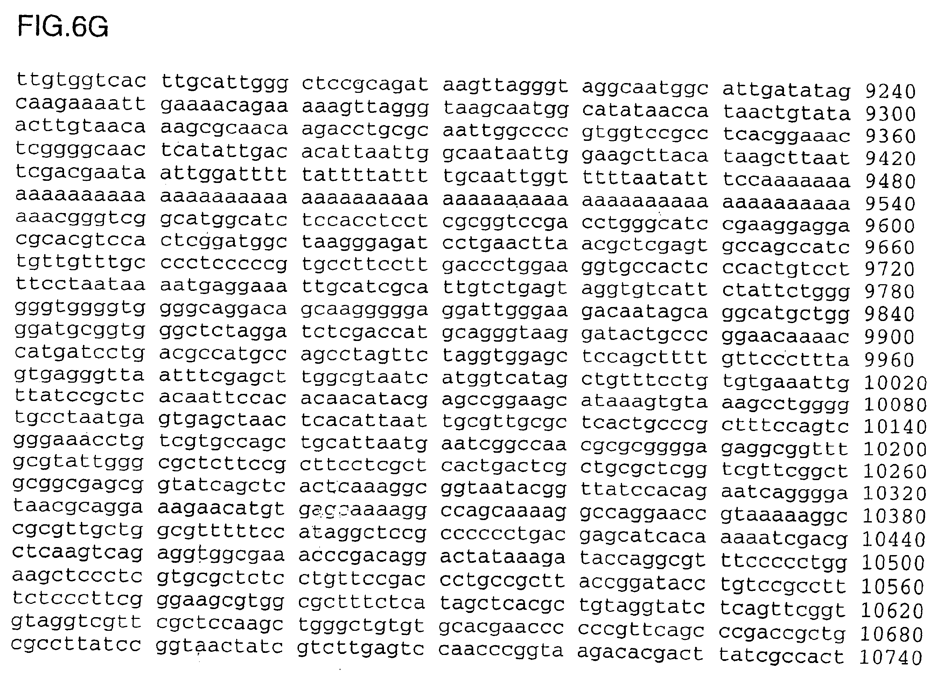

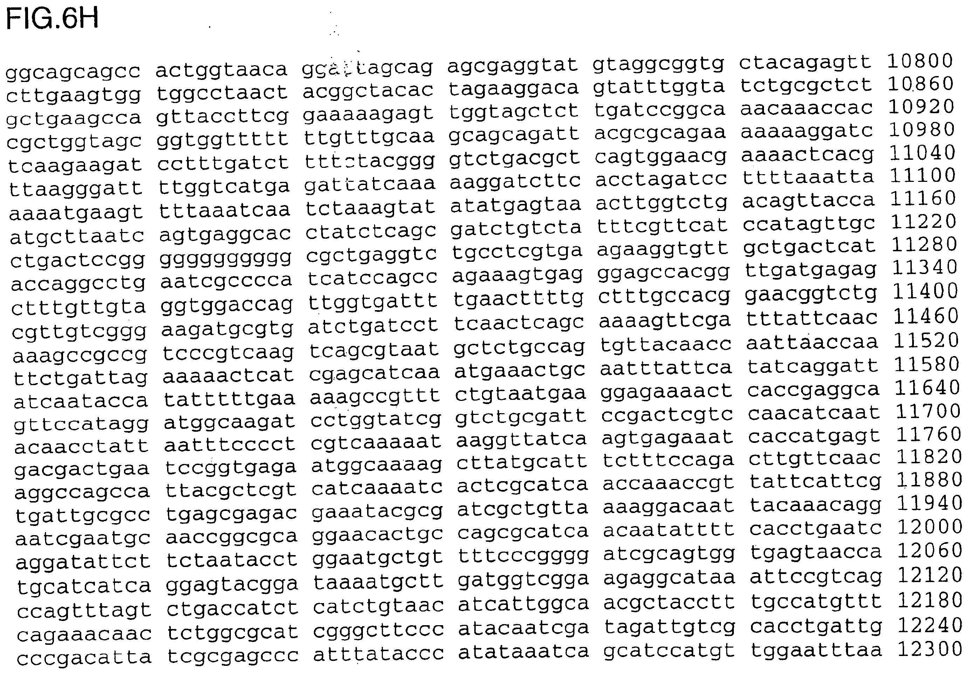

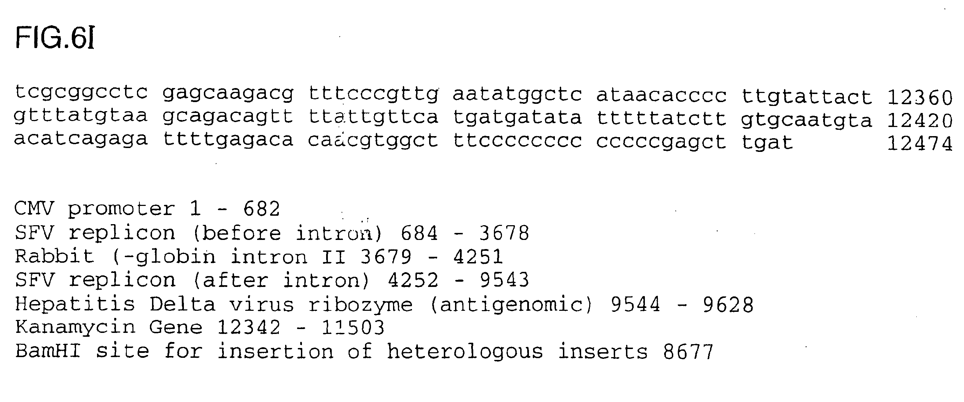

Figures 6A to 6D show the nucleotide sequence of plasmid pMP76 (SEQ ID no: 11, prepared as illustrated in Figures 8A to 8D;
Figure 7 illustrates subsections of plasmid pSFV link (see Figure 5);
Figure 8A to 8D show the construction of plasmid pMP76 from plasmids pMP53, pMP70, pMP47, pMP55 and pMP71;
Figures 9A to 9B show the construction of plasmids pMP53, pMP54 and pMP55 from plasmid pMP52;
Figure 10 shows the construction of plasmid MP52 from pUC19 using a linker sequence (SEQ ID no: 7, 8)
Figures 11A to 11B show the construction of plasmids pMP46, pMP47 and pMP70 from pUC19 and fragment from pSFV link, prepared as seen in Figure 7; and
Figures 12A to 12B show the construction of plasmid pMP71 from plasmid pCMV3.

### GENERAL DESCRIPTION OF INVENTION

As discussed above, the present invention provides a modified alphavirus DNA. The alphavirus preferably is Semliki Forest virus. In particular, the present invention provides a cloning vector for heterologous gene expression in a host, such as an animal or human.

The promoter sequence may comprise a promoter of eukaryotic or prokaryotic origin. Suitable promoters are the cytomegalovirus immediate early promoter (pCMV), although other promoters, such as the Rous sarcoma virus long-terminal repeat promoter (pRSV), since, in the case of these and similar promoters, transcription is performed by the DNA-dependent RNA polymerase of the host cell. Additionally, the SP6, T3 or T7 promoters can be used, provided that the cell has first been transformed with genes encoding SP6, T3 or T7 RNA polymerase molecules which are either inserted into the chromosome or remain episomal. Expression of these (SP6, T3, T7) RNA polymerase-encoding genes is dependent on the host cell DNA-dependent RNA polymerase.

The heterologous DNA insert may comprise the coding sequence for a desired product, which may be a biologically active protein or polypeptide, for example, the heterologous DNA insert may code for HIV sequences, e.g., an immunogenic or antigenic protein or polypeptide, or a therapeutically active protein or polypeptide. The heterologous DNA may also comprise additional sequences, such as a sequence complementary to an RNA sequence which is a self-cleaving ribozyme sequence.

The DNA vectors provided herein may be administered to a host, including a human host, for in vivo expression of the heterologous DNA sequence, in accordance with a further aspect of the invention, in order to generate an immune response in the host, which may be a protective immune response. The DNA vectors may be further formulated into immunogenic compositions for such administration.

### BIOLOGICAL DEPOSITS

Certain vectors that contain the Semliki Forest virus replicon and referred to herein have been deposited with the American Type Culture Collection (ATCC) located at 10801 University Boulevard, Manassas, VA 20110-2209, U.S.A., pursuant to the Budapest Treaty and prior to the filing of this application.

Samples of the deposited plasmids will become available to the public upon grant of a patent based upon this United States patent application and all restrictions on access to the deposits will be removed at that time. Non-viable deposits will be replaced. The invention described and claimed herein is not to be limited in scope by plasmids deposited, since the deposited embodiment is intended only as an illustration of the invention.

**Deposit Summary**

| Plasmid | ATCC Designation | Date Deposited |
|---|---|---|
| pMP76 | 203462 | 12^{th} Nov 1998 |

### EXAMPLES

The above disclosure generally describes the present invention. A more complete understanding can be obtained by reference to the following specific Examples. These Examples are described solely for purposes of illustration and are not intended to limit the scope of the invention. Changes in form and substitution of equivalents are contemplated as circumstances may suggest or render expedient. Although specific terms have been employed herein, such terms are intended in a descriptive sense and not for purposes of limitations.

Methods of molecular genetics, protein biochemistry and immunology used but not explicitly described in this disclosure and these Examples are amply reported in the scientific literature and are well within the ability of those skilled in the art.

### EXAMPLE 1

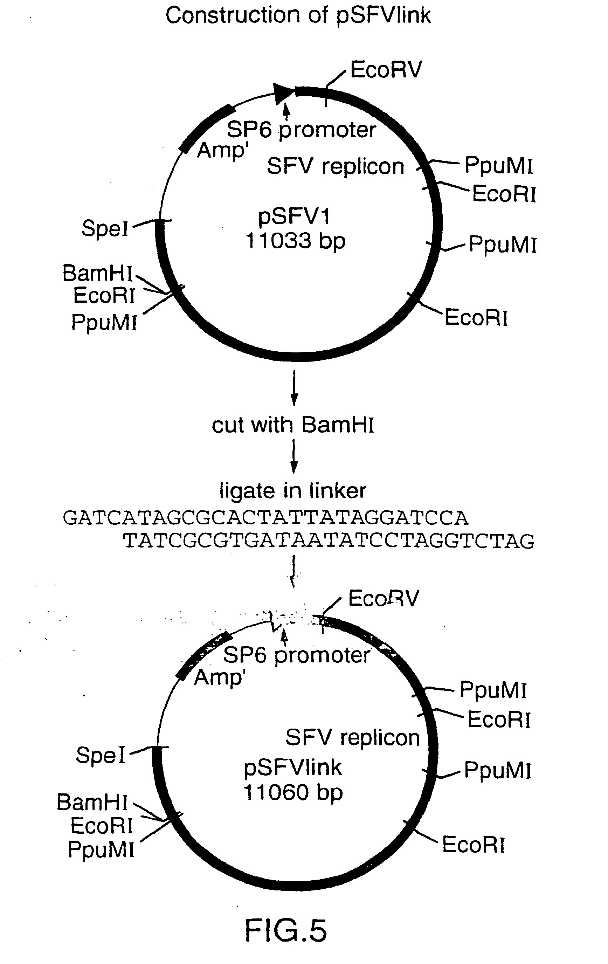

This Example describes the construction of plasmid pMP76 as outlined in Figures 5, 7, 8A, 8B, 8C, 8D, 9A, 9B, 10, 11A, 1113, 12A and 12B.

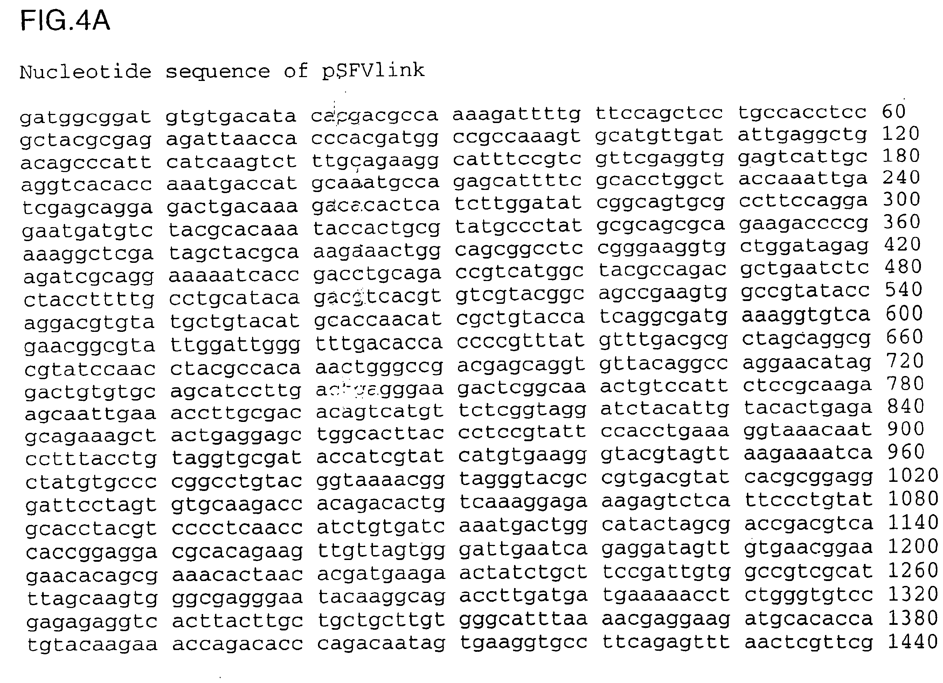

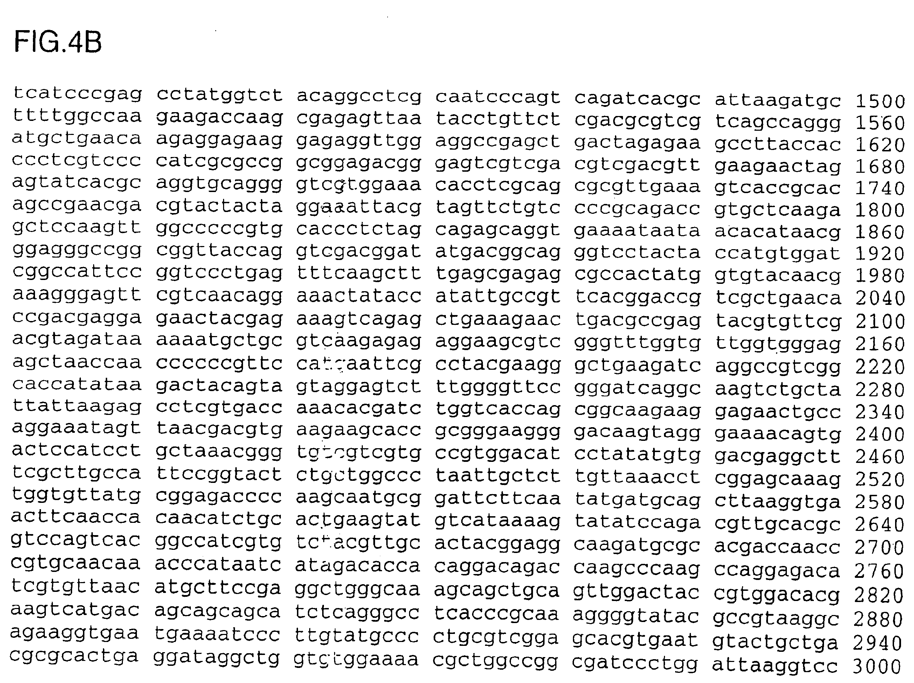

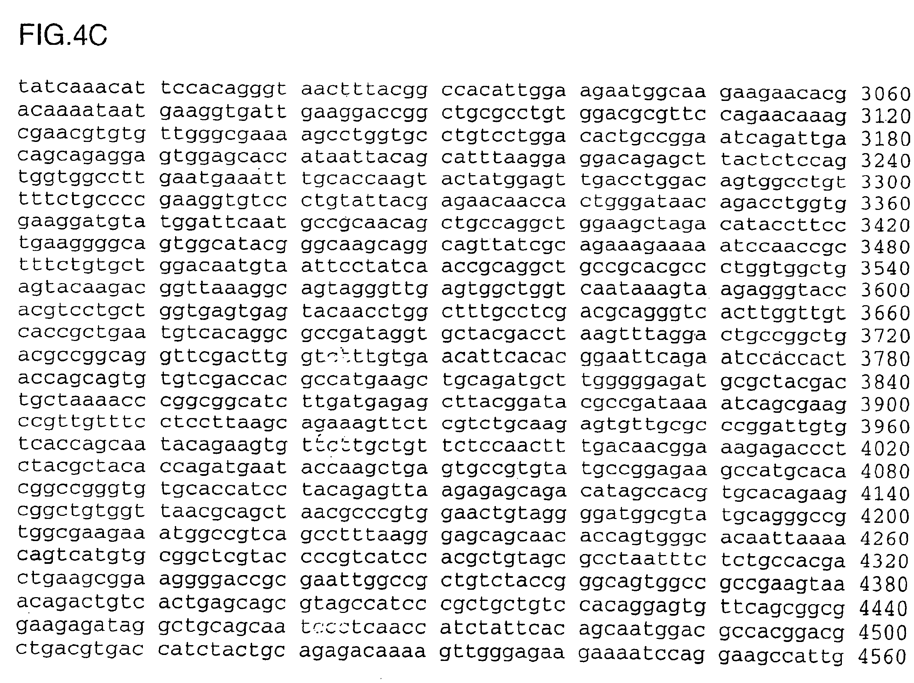

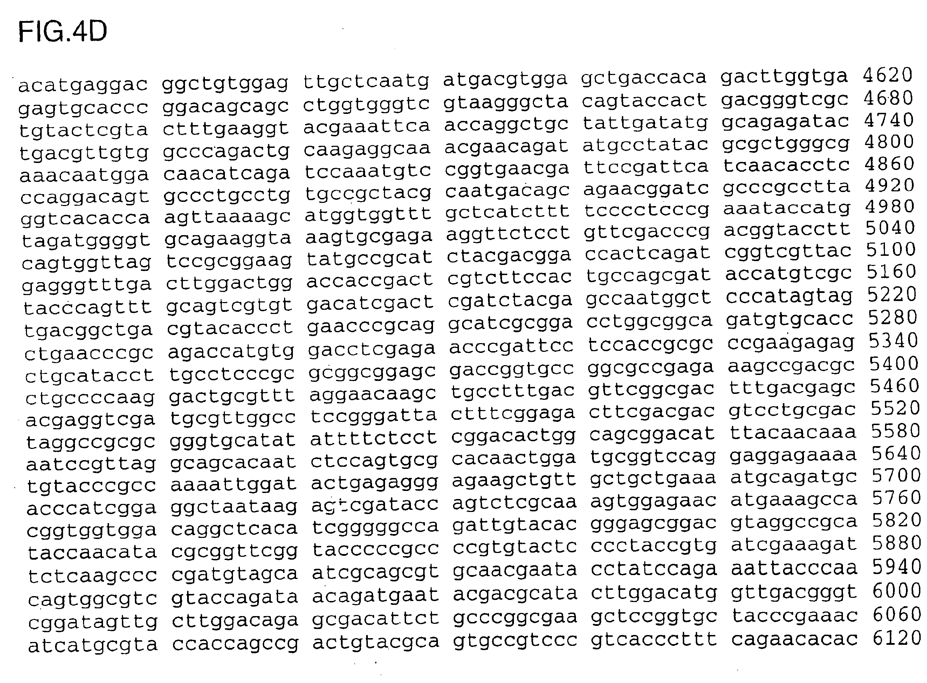

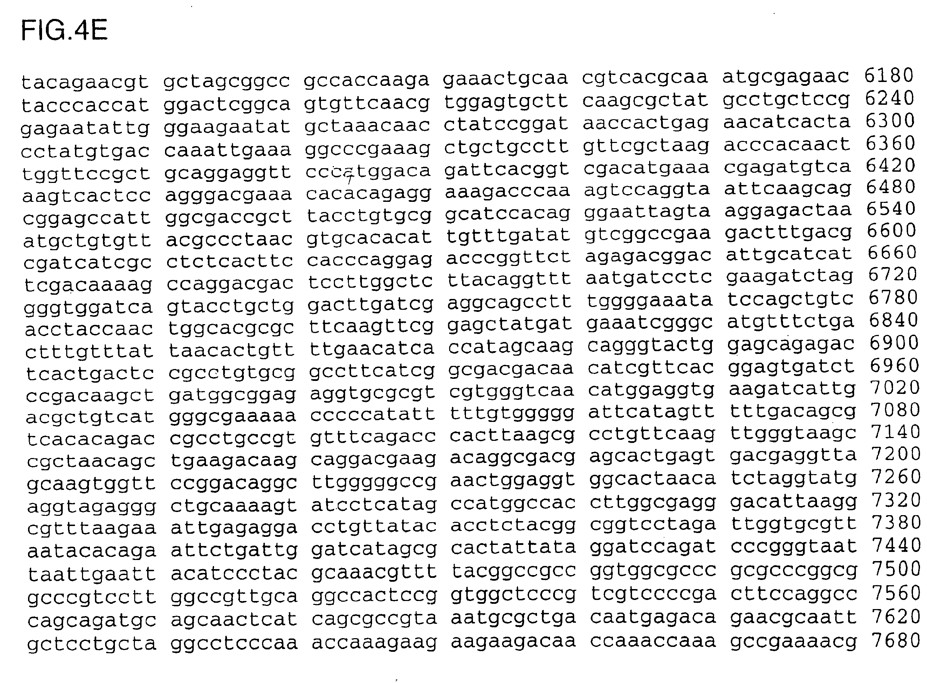

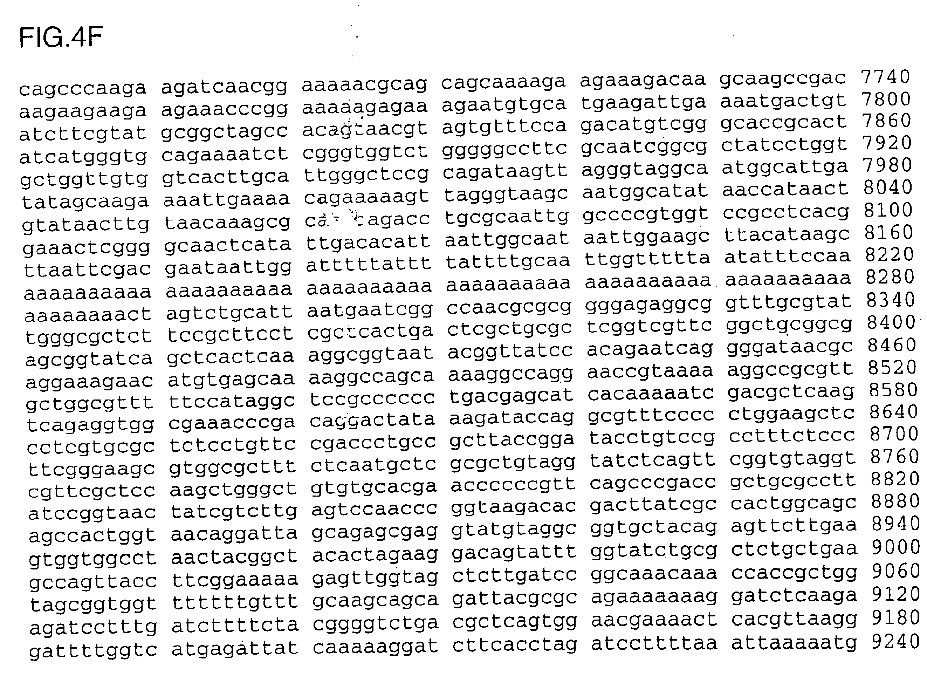

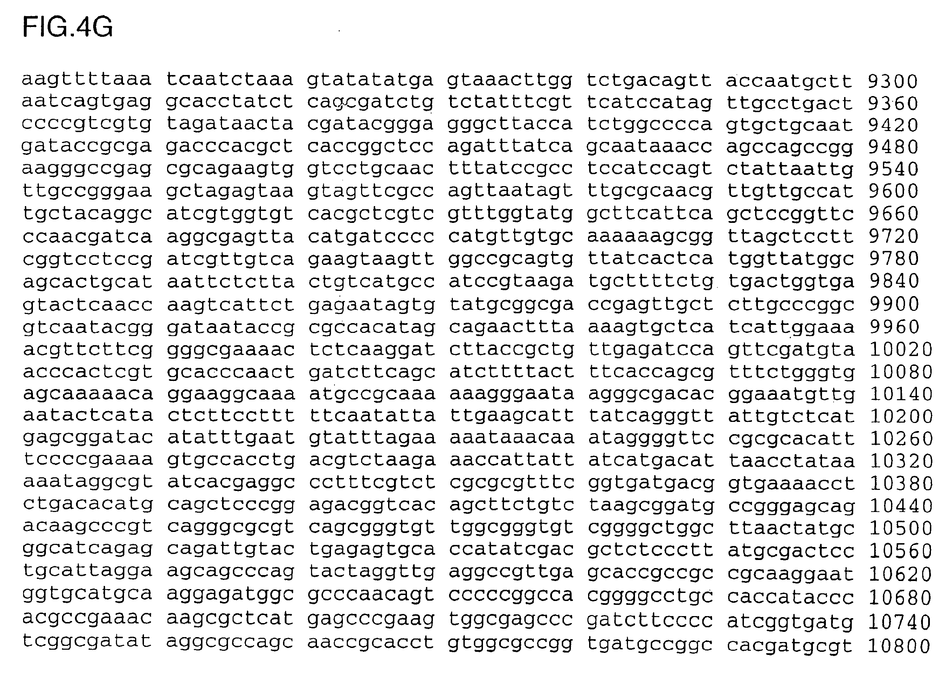

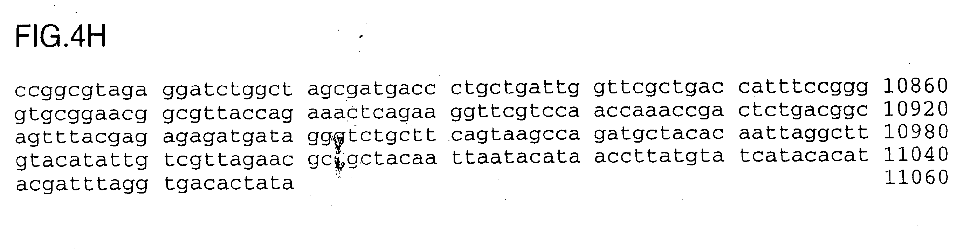

Plasmid pSFV link was created by restricting plasmid pSFV1 (Gibco) with BamHI. This plasmid was then ligated with a linker (SEQ ID no: 5 and 6) to produce plasmid pSFV link (Figures 4A to 4D, Figure 5).

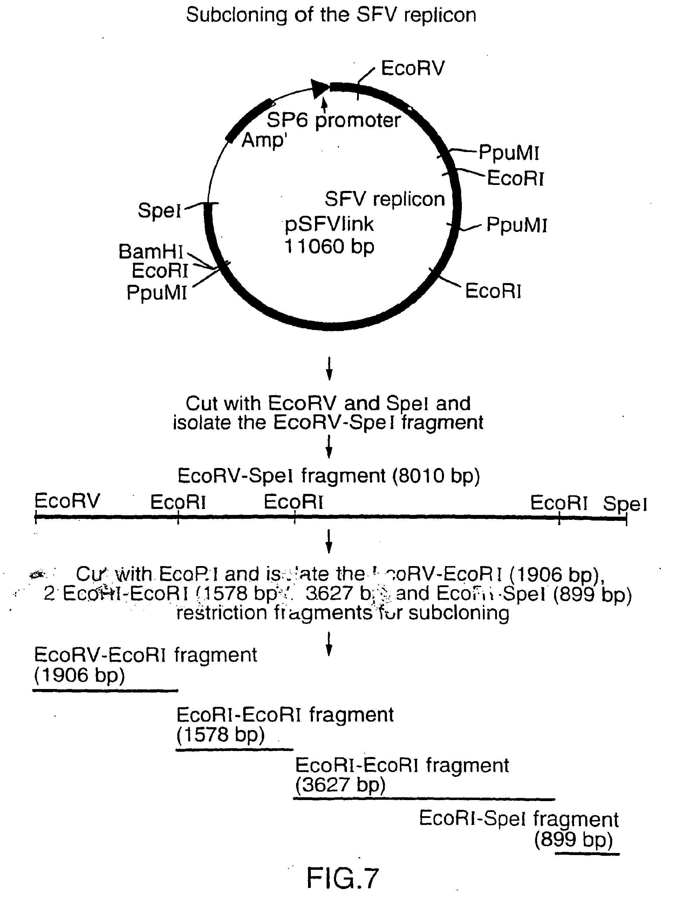

Some of the SFV replicon fragments were subcloned by restricting pSFVlink with EcoRV and SpeI and isolating the 890bp EcoRV-SpeI fragment. This fragment was then restricted with EcoRI and the 1906bp EcoRV-EcoRI, the 1578bp and 3627bp EcoRI-EcoRI and the 899bp EcoRI-SpeI fragments isolated (Fig.7).

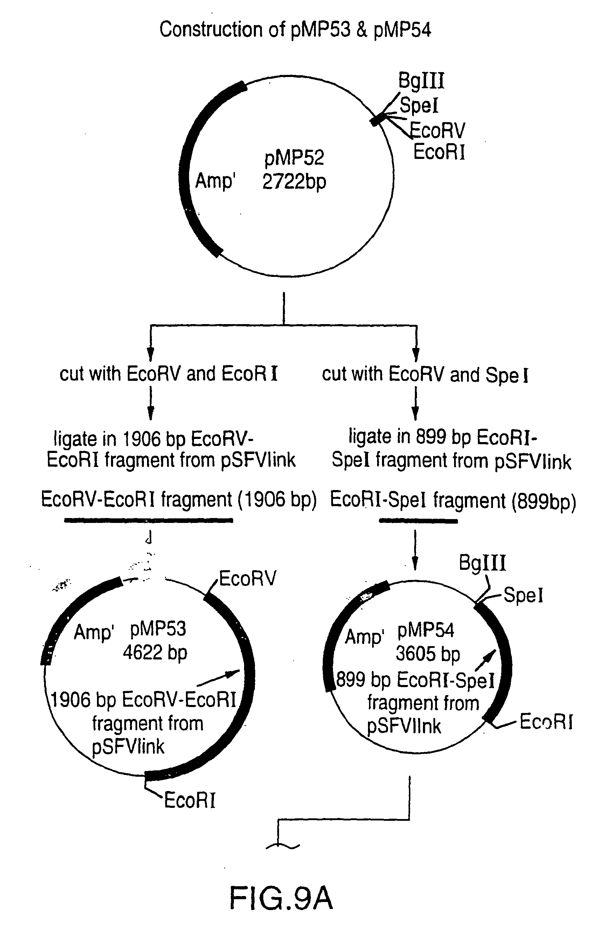

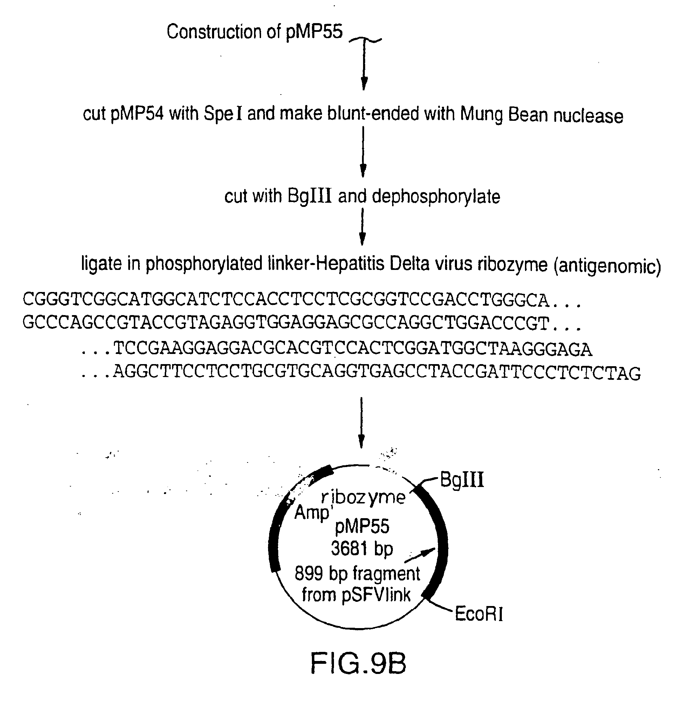

The 1909bp EcoRV-EcoRI SFV fragment was cloned into EcoRV-EcoRI restricted plasmid pMP52 to produce plasmid pMP53 (Fig.9A). The 899bp EcoRI-SpeI SFV fragment was cloned into EcoRI-SpeI restricted pMP52 to produce pMP54 (Fig.9A). Plasmid pMP54 was then restricted with SpeI and made blunt-ended with Mung Bean nuclease. The plasmid was then restricted with BglII, dephosphorylated and ligated to the hepatitis delta virus ribozyme linker (SEQ ID nos. 9 and 10), that had been phosphorylated, to produce pMP55 (Fig. 9B) .

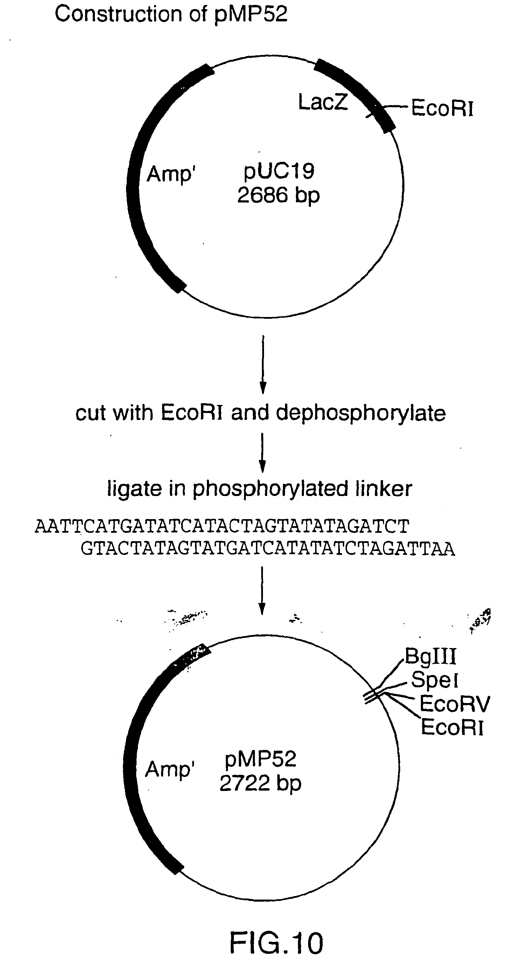

Plasmid pMP52 was created by ligating a linker (SEQ ID nos:7,8), into the EcoRI site of pUC19 (Fig.10).

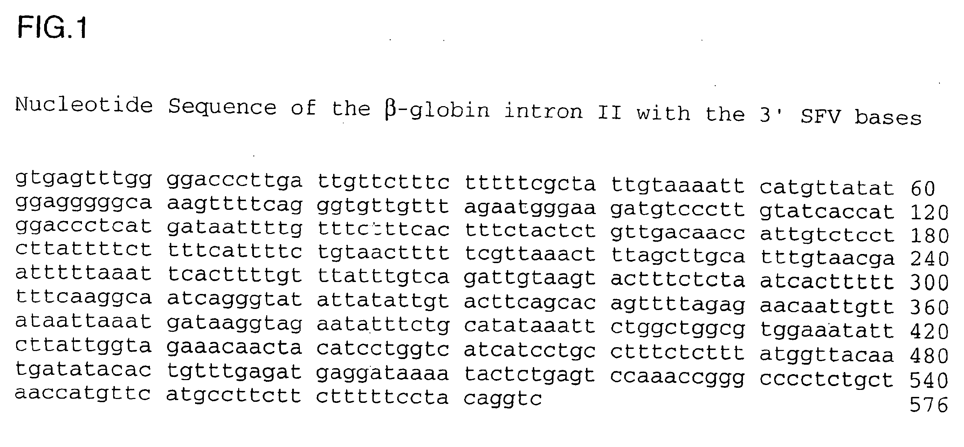

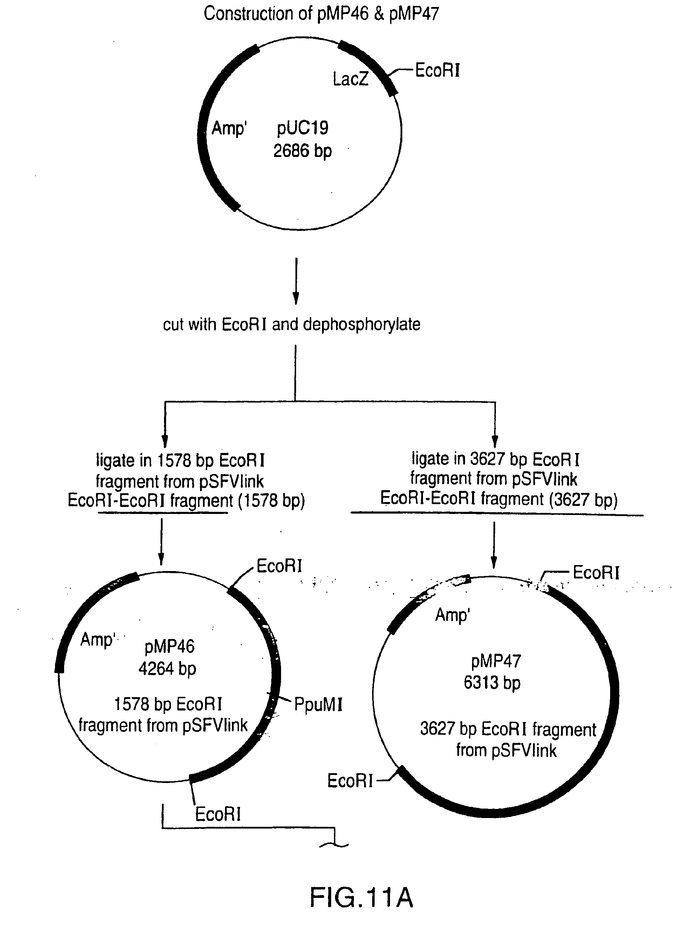

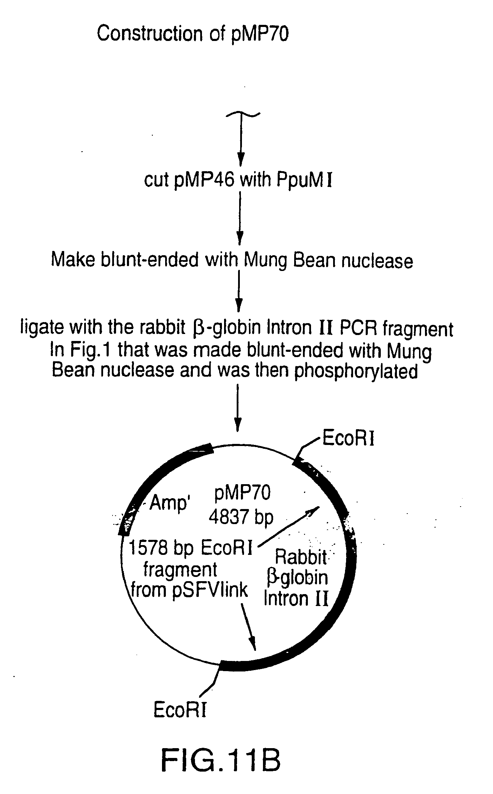

The 1578bp EcoRI-SFV fragment ws cloned into the EcoRI site of pUC19, to produce pMP46 (Fig.11A) . This plasmid was then restricted with PpuM1 and made blunt-ended with Mung Bean nuclease. The rabbit β-globin intron II PCR fragment (Fig. 1) was made blunt-ended with Mung Bean nuclease, phosphorylated and ligated to the PpuMI restricted pMP46 to produce plasmid pMP70 (Fig.11B).

The 3627bp EcoRI SFV fragment was cloned into the EcoRI site of pUC19 to produce pMP47 (Fig.11A).

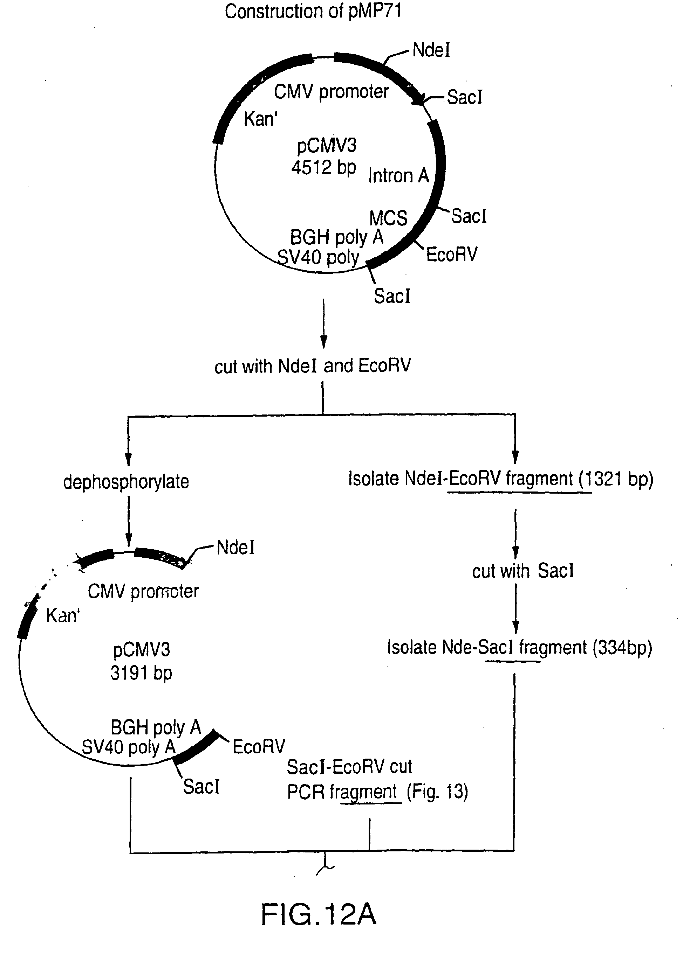

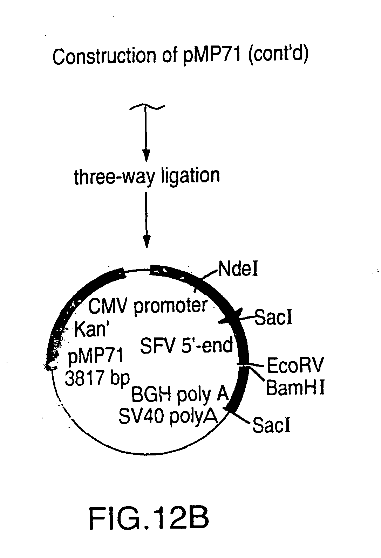

Plasmid pCMV3, which contains the CMV promoter, Intron A sequence, BGH poly A sequence and SU40 poly A sequence, was restricted with NdeI and EcoRV. The 3191bp NdeI-EcoRV fragment was isolated and dephosphorylated. The 1321bp NdeI-EcoRV fragment was isolated and restricted with SacI. The NdeI-SacI fragment of 334bp was isolated (Fig.12A). The isolated SacI-EcoRV PCR fragment containing the 5'-end of SFV was ligated to the previously isolated 334bp NdeI-SacI fragment and the 3191bp. NdeI-EcoRV fragment to produce pMP71 (Fig.12A and 12B).

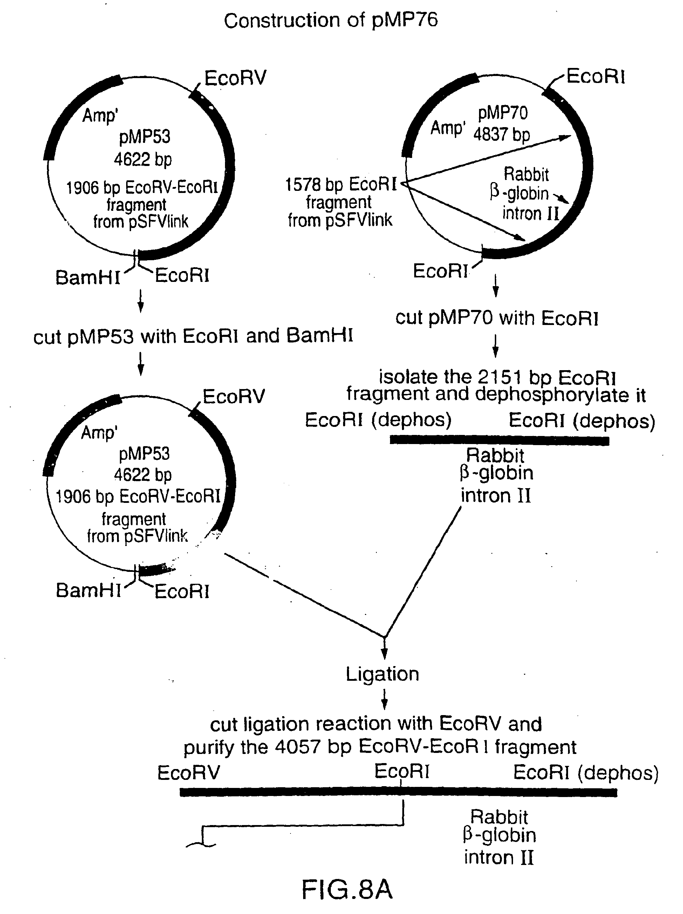

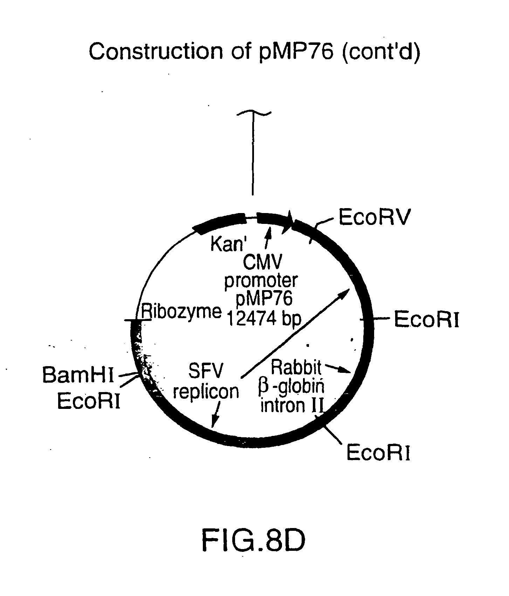

Plasmid pMP53 was then restricted with EcoRI and BamHI and ligated to the isolated and dephosphorylated 2151bp EcoRI fragment from pMP70 (Fig.8A). This ligation was then restricted with EcoRV and the 4057bp EcoRV-EcoRI fragment purified(Fig.8A).

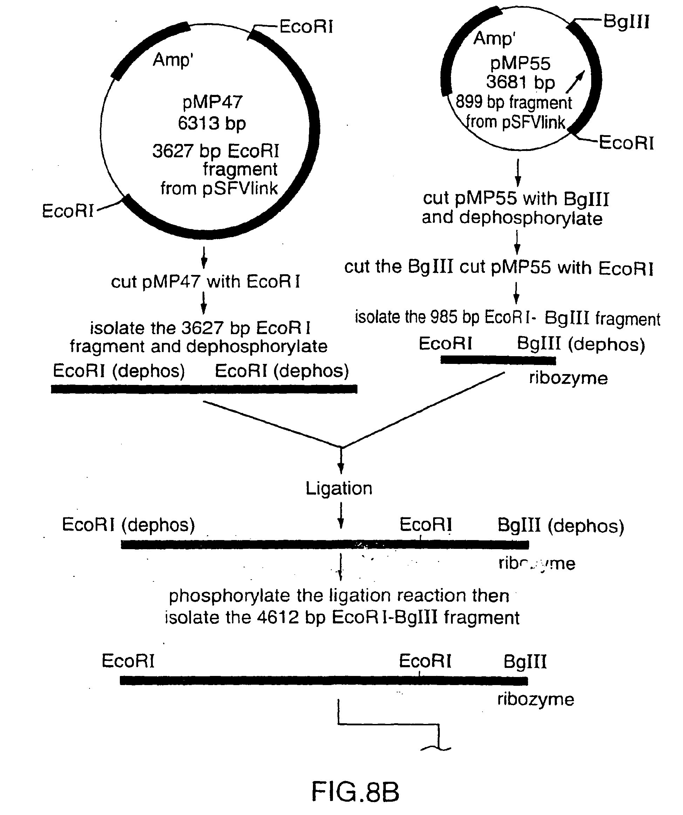

Plasmid pMP47 was restricted with EcoRI and the 3627bp EcoRI fragment isolated and dephosphorylated (Fig.8B). Plasmid pMP55 was then restricted with BglII, dephosphorylated and restricted with EcoRI. The 985bp EcoRI-BglII fragment was isolated and ligated to the previously isolated EcoRI fragment from pMP47 (Fig. 8B). The ligation reaction was then phosphorylated and the 4612bp EcoRI-BglII fragment isolated.

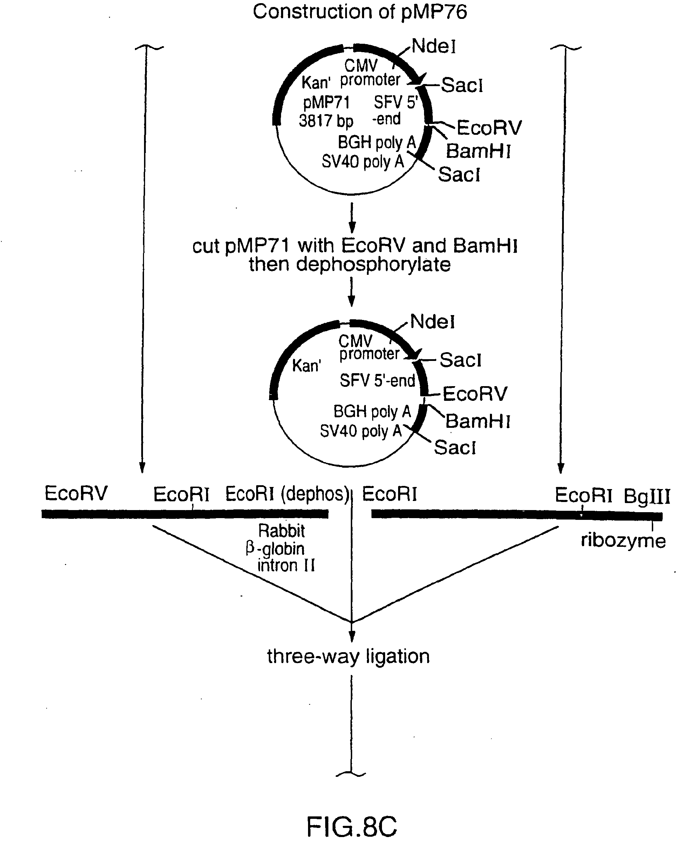

Plasmid pMP71 was restricted with EcoRV and BamHI then dephosphorylated. This fragment was used in a 3-way ligation with the previously isolated 4612bp EcoRI-SglII fragment from pMP47 and pMP55, and the 4057bp EcoRV-EcoRI fragment from pMP53 and pMP70, to produce pMP76 (Figs. 8B and 8C) .

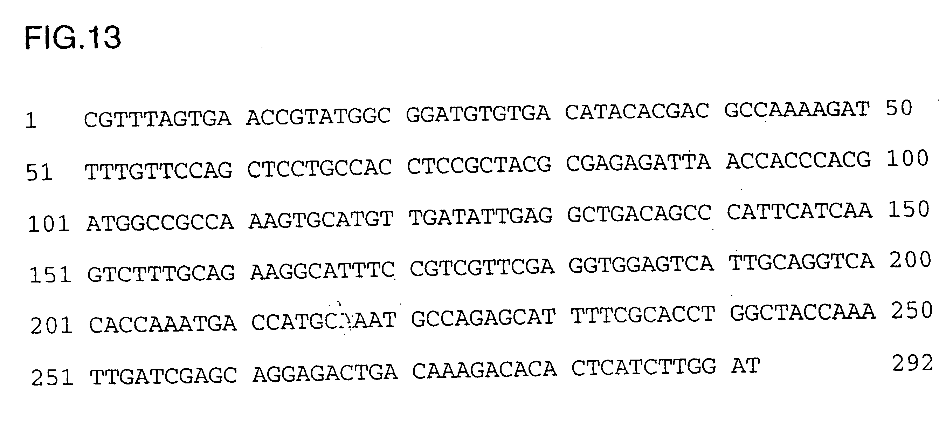

The 5' end of the SFV replicon was produced by PCR amplification of pSFVl using primers SFV-5'-3 having the sequence 5' -ATCTATGAGCTCGrrrAGTGAACCGTATGGCGGATGTGTGACATACA-3 . and EcoR-SPE having the sequence 5'-TCCACCTCCAAGGATATCCAAGATGAGTGTG-3' (SEQ ID no: 9 and SEQ ID no: 10 respectively) between the CMV promoter and the 5' end of the SFV replicon. The resulting PCR fragment was restricted with SacI and EcoRV (Fig. 13; SEQ ID no: 11) and the fragment isolated.

### SUMMARY OF DISCLOSURE

In summary of this disclosure, the present invention provides a modified alphavirus-based expression vector wherein at least one optimal splice site is introduced to the alphavirus replicon to prevent aberrant splicing of the alphavirus genome; and improve transport of RNA out of the nucleus.

### REFERENCES

1. Fulginiti, V.A., Eller, J.J., Sieber, O.F., Joyner, J.W., Minamitani, M. and Meiklejohn, G., (1969) Am. J. Epidemiol. 89 (4), 435-448.
2. Chin, J., Magoffin, R.L., Shearer, L.A., Schieble, J.H. and Lennette, E.H. (1969) Am. J. Epidemiol. 89 (4), 449-463.
3. Jensen, K.E., Peeler, B.E. and Dulworth, W.G. (1962) J. Immunol. 89, 216-226.
4. Murphy, B.R., Prince, G.A., Collins, P.L., Van Wyke-Coelingh, K., Olmstead, R.A., Spriggs, M.K., Parrott, R.H., Kim, H.-Y., Brandt, C.D. and Chanock, R.N. (1988) Vir. Res. 11, 1-15.
5. Chapman, B.S.; Thayer, R.M.; Vincent, K.A. and Haigwood, N.L., Nucl. Acids. Res. 1991, 19: 3979-3986.
6. Huang, Zhi-ming and Yen, T. S. Benedict, Molecular and Cell Biology, July 1995, p.3864-3869.

## Claims

1. An expression vector, comprising a DNA molecule which comprises the complement of the complete alphavirus RNA genome regions which are essential for replication of said alphavirus RNA and further comprises a heterologous DNA sequence capable of expression in a host, said heterologous DNA sequence being inserted into a region of the DNA molecule which is non-essential to replication thereof, and the DNA molecule being placed under transcriptional control of a promoter sequence functional in said host, wherein at least one heterologous intron is inserted into the alphavirus replicon sequence at a location which generates perfect splice junctions and which precludes replication of the alphavirus vector unless the intron is authentically removed by splicing.

2. The vector claimed in claim 1, wherein said promoter is placed upstream of the 5'-end of the DNA molecule such that the resultant transcript has an authentic 5'-end.

3. The vector claimed in claim 1 or 2, wherein said promoter is the cytomegalovirus immediate early promoter.

4. The vector as claimed in any one of claims 1 to 3 which further comprises an additional DNA sequence at the 3'-end of the DNA molecule and complementary to an RNA sequence which is a self-cleaving ribozyme.

5. The vector as claimed in claim 4, wherein the additional DNA sequence is a DNA sequence complementary to a hepatitis delta virus ribozyme sequence.

6. The vector claimed in any one of claims 1 to 5, wherein the heterologous intron is provided by the DNA sequence of the rabbit β-globin intron II.

7. The vector claimed in any one of claims 1 to 6, wherein the alphavirus is a Semliki Forest virus and at least one heterologous splice site being located at a Ppu-Ml site at position 2719 and/or at a Pvull site at position 2518, 3113, 6498 and/or 6872 within an EcoRV-Spel fragment of the SFV replicon, as shown in Figure 3.

8. A cloning vector suitable for expression in a host cell of an heterologous DNA sequence, which comprises:
a DNA molecule which comprises the complement of the complete alphavirus RNA genome replication regions and has a cloning site for insertion therein of a heterologous DNA sequence capable of expression in a host cell, said cloning site being located in a region of the DNA molecule which is non-essential to replication thereof;
a promoter sequence functional in said host cell and transcriptionally controlling said DNA molecule, said promoter sequence being placed upstream of the 5'-end of the DNA molecule such that the resultant transcript has an authentic 5'-end;
at least one heterologous intron which is provided in the complement of the DNA molecule and is inserted into the alphavirus replicon sequence at a location which generates perfect splice junctions and which precludes replication of the alphaviurs vector unless the intron is authentically removed by splicing; and
an additional DNA sequence at the 3'-end of the DNA molecule and complementary to an RNA sequence which is a self-cleaving ribozyme.

9. The cloning vector claimed in claim 8, wherein said alphavirus is a Semliki Forest virus and wherein the at least one heterologous splice site is located at the Ppu-MI site at position 2719 and/or at a Pvull site at position 2518, 3113, 6498 and/or 6872 within an EcoRV-Spel fragment of the SFV replicon, as shown in Figure 3.

10. The cloning vector as claimed in claim 8 or 9, wherein the additional DNA sequence is a DNA sequence complementary to a hepatitis delta virus ribozyme sequence.

11. The cloning vector claimed in any one of claims 8 to 10 which is plasmid pMP76 (ATCC Accession No. 203462).

## Patentansprüche

1. Ein Expressionsvektor, der ein DNA-Molekül umfasst, welches das Komplement der vollständigen Alphavirus-RNA-Genomregionen umfasst, die für die Replikation dieser Alphavirus-RNA essentiell sind und außerdem eine heterologe DNA-Sequenz umfasst, die in der Lage ist, in einem Wirt exprimiert zu werden, wobei diese heterologe DNA-Sequenz in eine Region des DNA-Moleküls eingefügt ist, die nicht-essentiell für dessen Replikation ist, und wobei das DNA-Molekül unter die Transkriptionskontrolle einer in diesem Wirt funktionalen Promotorsequenz gestellt ist, wobei mindestens ein heterologes Intron in die Alphavirus-Replikonsequenz an einem Ort eingefügt ist, wodurch perfekte Spleissstellen entstehen und wodurch die Replikation des Alphavirus-Vektors verhindert wird, es sei denn, das Intron wird tatsächlich durch Spleissen entfernt.

2. Der Vektor, der in Anspruch 1 beansprucht wird, wobei der Promotor sich upstream von dem 5'-Ende des DNA-Moleküls befindet, so dass das resultierende Transkript ein echtes 5'-Ende aufweist.

3. Der Vektor, der in Anspruch 1 oder 2 beansprucht wird, wobei der Promotor der immediate/early-Promotor des Zytomegalievirus ist.

4. Der Vektor, wie er in einem der Ansprüche 1 bis 3 beansprucht wird, der außerdem eine zusätzliche DNA-Sequenz am 3'-Ende des DNA-Moleküls umfasst, die komplementär ist zu einer RNA-Sequenz, die ein selbst-spleissendes Ribozym ist.

5. Der Vektor, wie er in Anspruch 4 beansprucht wird, wobei die zusätzliche DNA-Sequenz eine DNA-Sequenz ist, die komplementär zu einer Ribozym-Sequenz des Heptatis-delta-Virus ist.

6. Der Vektor, der in einem der Ansprüche 1 bis 5 beansprucht wird, wobei das heterologe Intron durch die DNA-Sequenz des Hasen-β-Globin-Introns II bereitgestellt wird.

7. Der Vektor, der in einem der Ansprüche 1 bis 6 beansprucht wird, wobei der Alphavirus ein Simliki Forest-Virus ist und mindestens eine heterologe Spleissstelle sich an der Ppu-MI-Restriktionsstelle an Position 2719 und/oder an einer PvuII-Restriktionsstelle an Position 2518, 3113, 6498 und/oder 6872 innerhalb eines EcoRV-SpeI-Fragments des SFV-Replikons befindet, wie in Figur 3 dargestellt.

8. Ein Klonierungsvektor, der für die Expression einer heterologen DNA-Sequenz in einer Wirtszelle geeignet ist, umfassend:
ein DNA-Molekül, das das Komplement der vollständigen Alphavirus-RNA-Genomreplikationsregionen umfasst und eine Klonierungsstelle aufweist für die Insertion einer heterologen DNA-Sequenz, die in der Lage ist, in einer Wirtszelle exprimiert zu werden, wobei diese Klonierungsstelle sich in einer Region des DNA-Moleküls befindet, die nicht-essentiell für dessen Replikation ist;
eine in dieser Wirtszelle funktionale Promotorsequenz, die die Transkription dieses DNA-Moleküls kontrolliert, wobei diese Promotorsequenz sich upstream von dem 5'-Ende des DNA-Moleküls befindet, so dass das resultierende Transkript ein echtes 5'-Ende aufweist;
mindestens ein heterologes Intron, das in dem Komplement des DNA-Moleküls vorliegt und in die Alphavirus-Replikonsequenz an einem Ort eingefügt ist, wodurch perfekte Spleissstellen entstehen und wodurch die Replikation des Alphavirus-Vektor verhindert wird, es sei denn, das Intron wird in der Tat durch Spleissen entfernt; und
eine zusätzliche DNA-Sequenz am 3'-Ende des DNA-Moleküls, die komplementär ist zu einer RNA-Sequenz, die ein selbst-spleissendes Ribozym ist.

9. Der Klonierungsvektor, der in Anspruch 8 beansprucht wird, wobei der Alphavirus ein Semliki Forest-Virus ist und wobei mindestens eine heterologe Spleissstelle sich an der Ppu-MI-Restriktionsstelle an Position 2719 und/oder an einer PvuII-Restruktionsstelle an Position 2518, 3113, 6498 und/oder 6872 innerhalb eines EcoRV-SpeI-Fragments des SFV-Replikons befindet, wie in Figur 3 dargestellt.

10. Der Klonierungsvektor, wie er in Anspruch 8 oder 9 beansprucht wird, wobei die zusätzlich DNA-Sequenz eine DNA-Sequenz ist, die komplementär zu einer Ribozym-Sequenz des Hepatits-delta-Virus ist.

11. Der Klonierungsvektor, der in einem der Ansprüche 8 bis 10 beansprucht wird, der das Plasmid pMP76 (ATCC Zugangsnr. 203462) ist.

## Revendications

1. Vecteur d'expression, comprenant une molécule d'ADN qui comprend le complémentaire des régions complètes du génome ARN d'un alphavirus qui sont essentielles pour la réplication dudit ARN d'alphavirus et qui comprend en outre une séquence d'ADN hétérologue capable d'une expression chez un hôte, ladite séquence d'ADN hétérologue étant insérée dans une région de la molécule d'ADN qui est non essentielle à la réplication de celle-ci, et la molécule d'ADN étant placée sous le contrôle transcriptionnel d'une séquence promotrice fonctionnelle dans ledit hôte, dans lequel au moins un intron hétérologue est inséré dans la séquence d'un réplicon d'alphavirus au niveau d'un emplacement qui génère des jonctions d'épissage parfaites et qui empêche la réplication du vecteur d'alphavirus sauf si l'intron est retiré véritablement par épissage.

2. Vecteur selon la revendication 1, dans lequel ledit promoteur est placé en amont de l'extrémité 5' de la molécule d'ADN de sorte que le transcrit résultant ait une extrémité 5' véritable.

3. Vecteur selon la revendication 1 ou 2, dans lequel ledit promoteur est le promoteur précoce immédiat du cytomégalovirus.

4. Vecteur selon l'une quelconque des revendications 1 à 3, qui comprend en outre une séquence d'ADN supplémentaire à l'extrémité 3' de la molécule d'ADN et complémentaire à une séquence d'ARN qui est un ribozyme autoclivable.

5. Vecteur selon la revendication 4, dans lequel la séquence d'ADN supplémentaire est une séquence d'ADN complémentaire à une séquence de ribozyme du virus delta de l'hépatite.

6. Vecteur selon l'une quelconque des revendications 1 à 5, dans lequel l'intron hétérologue est fourni par la séquence d'ADN de l'intron II de la β-globine du lapin.

7. Vecteur selon l'une quelconque des revendications 1 à 6, dans lequel l'alphavirus est un virus de la forêt Semliki et au moins d'un site d'épissage hétérologue est situé au niveau d' un site Ppu-MI en position 2719 et/ou un site PvuII en position 2518, 3113, 6498 et/ou 6872 dans un fragment EcoRV-SPeI du réplicon de SFV, tel que montré dans la figure 3.

8. Vecteur de clonage approprié pour l'expression dans une cellule hôte d'une séquence d'ADN hétérologue, qui comprend :
une molécule ADN qui comprend le complémentaire des régions complètes de réplication du génome ARN d'un alphavirus et qui a un site de clonage pour une insertion à l'intérieur de celle-ci d'une séquence d'ADN hétérologue capable d'une expression dans une cellule hôte, ledit site de clonage étant situé dans une région de la molécule d'ADN qui est non essentielle à la réplication de celle-ci ;
une séquence promotrice fonctionnelle dans ladite cellule hôte et contrôlant de manière transcriptionnelle ladite molécule d'ADN, ladite séquence promotrice étant placée en amont de l'extrémité 5' de la molécule d'ADN de sorte que le transcrit résultant ait une extrémité 5' véritable,
au moins un intron hétérologue qui est fourni dans le complémentaire de la molécule d'ADN et qui est inséré dans la séquence de réplicon d'un alphavirus au niveau d'un emplacement qui génère des jonctions d'épissage parfaites et qui empêche la réplication du vecteur d'alphavirus sauf si l'intron est retiré véritablement par épissage ; et
une séquence d'ADN supplémentaire à l'extrémité 3' de la molécule d'ADN et complémentaire à une séquence d'ARN qui est un ribozyme autoclivable.

9. Vecteur de clonage selon la revendication 8, dans lequel ledit alphavirus est un virus de la forêt Semliki et dans lequel le site d'épissage hétérologue est situé au niveau du site Ppu-MI en position 2719 et/ou d'un site PvuII en position 2518, 3113, 6498 et/ou 6872 dans un fragment EcoRV-SPel du réplicon de SFV, tel que montré dans la figure 3.

10. Vecteur de clonage selon la revendication 8 ou 9, dans lequel la séquence d'ADN supplémentaire est une séquence d'ADN complémentaire à une séquence de ribozyme du virus delta de l'hépatite.

11. Vecteur de clonage selon l'une quelconque des revendications 8 à 10 qui est le plasmide pMP76 (No. d'accès ATCC 203462).
